# EUROPEAN PATENT SPECIFICATION

(11) **EP 2 370 115 B1**
(45) Date of publication and mention of the grant of the patent: **03.08.2016**
(21) Application number: 09796100.7
(22) Date of filing: 06.12.2009
(51) Int. Cl.: A61L 27/56, A61K 41/00, A61K 9/00, A61B 17/12

(54) **HYDROGEL SPONGES, METHODS OF PRODUCING THEM AND USES THEREOF**
HYDROGEL-SCHWÄMME, VERFAHREN ZU IHRER HERSTELLUNG UND IHRE VERWENDUNG
EPONGES D'HYDROGEL, LEURS PROCEDES DE PRODUCTION ET LEURS UTILISATION

(30) Priority: 04.12.2008 US 193505 P
(43) Date of publication of application: 05.10.2011
(73) Proprietor: Technion Research & Development Foundation Ltd., 32000 Haifa (IL)
(72) Inventor: KAULLY, Tamar, 17940 Adi (IL); LEVENBERG, Shulamit, 20186 Moreshet (IL)
(74) Representative: ABG Patentes, S.L.
(86) International application number: PCT/IL2009/001151
(87) International publication number: WO 2010/064251

(56) References cited:
- WO-A1-2005/061018
- US-A- 5 514 378
- US-A1- 2002 022 673
- US-A1- 2002 192 889
- US-A1- 2004 026 811
- A. SANNINO ET AL: "Synthesis and characterization of macroporous poly(ethylene glycol)-based hydrogels for tissue engineering application", JOURNAL OF BIOMEDICAL MATERIALS RESEARCH. PART A, vol. 79A, no. 2, 1 November 2006 (2006-11-01), pages 229-236, XP055240416, HOBOKEN, NY, US ISSN: 1549-3296, DOI: 10.1002/jbm.a.30780

## Description

### FIELD OF THE INVENTION

The present invention relates to hydrogel sponges and methods of producing and using same.

### BACKGROUND OF THE INVENTION

The development of microvascular networks is essential for long term viability of thick three dimensional (3D) tissue constructs *in vitro* and their *in-vivo* integration (Ford et al., 2006). As reported previously, addition of fibroblasts to a co-culture of human umbilical vein endothelial cells (HUVEC) and myoblasts promotes lumen structures development in porous biodegradable polymer scaffolds (Levenberg et al., 2005). Owing to their mechanical stability and controlled pore architecture, such scaffolds are advantageous for providing physical support for cell attachment and growth. Natural biosynthetic materials such as protein-conjugated polymeric hydrogels are usually more favorable to site-specific cell adhesion with the scaffold. Also, they are well suited for the transport of soluble factors, nutrients and waste materials due to their highly water-saturated nature (Ford et al., 2006).

However, one of the challenges with hydrogels has been to obtain controlled pore architectures (Ford et al., 2006). Several methods for producing macropore hydrogel sponges were applied addressing this challenge: casting hydrogel solution around a salt leached poly(lactic-co-glycolic acid) (PLGA) scaffold (Ford et al., 2006); solvent casting/salt leaching of poly(ethylene glycol) (PEG) di-acrylate (DA) (PEG-DA), and poly(ε-caprolactone) DA (PCL-DA) mixture (Park et al., 2007), PEG crosslinked with hydrolysable polyrotaxane (Lee et al., 2003), or salt leaching of poly(N-isopropylacrylamide) and poly(D,L-lactic acid) DA crosslinked with dextran segments (Huang et al., 2007).

PEG-Fibrinogen (PEG-Fib) was reported earlier to support cell culture. PEG-DA was shown to be an efficient crosslinker for altering the mechanical, physical and chemical properties of the PEG-Fib based hydrogel [Dikovsky et al., 2006, 2008; and WO 2005/055800; WO 2005/061018, WO 2008/126092].

U.S. Patent No. 5,514,378 teaches biocompatible porous polymer membranes which are prepared by dispersing salt particles in a biocompatible synthetic polymer solution. The solvent in which the synthetic polymer is dissolved is evaporated to produce a polymer/salt composite membrane. The polymer can then be heated and cooled at a predetermined constant rate to provide the desired amount of crystallinity. Salt particles are leached out of the membrane by immersing the membrane in water or another solvent for the salt but not the polymer. The membrane is dried, resulting in a porous, biocompatible membrane to which dissociated cells can attach and proliferate. A three-dimensional structure can be manufactured using the polymer membranes by preparing a contour drawing of the shape of the structure, determining the dimensions of thin cross-sectional layers of the shape, forming porous polymer membranes corresponding to the dimensions of the layers, and laminating the membranes together to form a three-dimensional matrix having the desired shape.

U.S. Patent No. 6,511,650 teaches a process for generating porous hydrogel materials by first creating gas pockets in the gel and then removing this gas. The removal of the gas creates a porous material, and the initial incorporation of sufficient gas allows one to create a material with an open, interconnected pore structure.

U.S. Patent Publication No. 2003/0040113 teaches a multi-layered system for tissue engineering, the system comprising: i) a cellular support layer configured to permit growth of cells in three-dimensions, the cellular support layer having an integrative side and a functional side, and wherein the cellular support is seeded with at least one cell type to create seeded cells; ii) an integration layer contacting the integrative side of the cellular support layer, wherein the integration layer is configured to be insertable into tissue; and iii) a protective layer contacting the functional side of the cellular support layer, the protective layer configured to protect the seeded cells from the effects of blood-borne agents and into the tissues of a patient.
A sol-gel solution is used to introduce cells into the matrix using capillary force.

Macroporous poly(ethylene glycol)-based hydrogels for tissue engineering applications using a foaming process have been described (Sannino et al., 2006).

U.S. Patent No. 2004/0026811 and U.S. Patent No. 2002/0022673 teach processes involving leaching of salts for preparing a macroporous scaffold for tissue engineering.

U.S. Patent Publication No. 2002/0192289 teaches a method of cancer treatment based on using a solid polymer gel to completely block blood vessels of tumor. A polymer aqueous solution is injected into blood vessels and forms a solid gel in blood vessels of tumor by applying electromagnetic radiation or temperature source at tumor tissue to induce crosslinking or phase transition.

PCT Publication WO 2004/031371 teaches a scaffold, which is a three-dimensional scaffold having interconnected pores and biologically active molecules physically entrapped therein. The scaffold is a lyophilized hydrogel of a biological or synthetic polymer. Pores are formed within the scaffold using freeze-dry lyophilization.

### SUMMARY OF THE INVENTION

It is an object of the present invention to provide macroporous hydrogel sponges.

It is another object of the present invention to provide such macroporous sponges which are produced in aqueous medium and are thus substantially or entirely devoid of organic solvents.

These and other objects of the invention are obtained by producing the macroporous sponges of the invention using the salt leaching method.

The present invention relates to a macroporous hydrogel sponge selected from: (i) a synthetic polymer hydrogel sponge, and (ii) a synthetic polymer-polypeptide conjugate hydrogel sponge, said macroporous hydrogel sponge being at least 20% porous and having a pore diameter of 50-1000 µm, wherein said synthetic polymer is crosslinked to an extent determined by effecting the crosslinking of the synthetic polymer or synthetic polymer-polypeptide conjugate in the presence of at least about 30 % by weight crosslinking agent.

In certain embodiments, the synthetic polymer is a polyalkylene glycol, preferably polyethylene glycol (PEG) functionalized at the terminal ends, preferably PEG-diacrylate (PEG-DA) or PEG-multiacrylate.

In certain embodiments, the polypeptide for use in the synthetic polymer-peptide conjugate of the invention is an extracellular matrix protein, preferably fibrinogen.

In certain embodiments, the crosslinking agent is a synthetic polymer, preferably PEG-DA. In some embodiments, the synthetic polymer and the crosslinking agent are identical. The crosslinking of the polypeptide-uncrosslinked synthetic polymer conjugate may be effected in the presence of at least about 30%, 40 %, 50%, 60%, 70%, 80%, 90%, or 95% by weight crosslinking agent.

In some embodiments, the hydrogel sponge of the invention is at least 50 % porous, wherein at least 20 % of said pores have a pore diameter of 50-1000, preferably, 100-600 µm.

In certain preferred embodiments of the invention, the hydrogel sponge consists solely of the crosslinked synthetic polymer, more preferably PEG-DA. In other preferred embodiments, the hydrogel sponge consists of a synthetic polymer-polypeptide conjugate hydrogel sponge, more preferably crosslinked PEG-DA-fibrinogen.

In certain embodiments, the hydrogel sponge of the invention may further comprise cells. In other certain embodiments, the hydrogel sponge may comprise one or more nonreactive agents attached to, coated on, embedded or impregnated in the sponge.

The hydrogel sponge of the invention may be provided in dry form. The dry sponge may be wetted before use.

In certain embodiments, the invention relates to a cosmetic or pharmaceutical composition comprising a macroporous hydrogel sponge as described hereinabove. The composition may comprise the hydrogel sponge in dry form suitable for implantation or for injection after wetting.

In certain embodiments, the invention further relates to the use of the macroporous hydrogel sponge or a cosmetic or pharmaceutical composition comprising it as scaffolds in tissue engineering for tissue repair.

In certain embodiments, the invention still further relates to the use of a synthetic polymer macroporous hydrogel sponge of the invention or a pharmaceutical composition comprising it for blocking blood vessels, for example, of tumors, optionally in combination with a chemotherapeutic or antiangiogenic agent loaded on the hydrogel.

The present invention further relates to a method of treating a tissue defect in a subject in need thereof, the method comprising administering to the subject a macroporous hydrogel sponge described herein, thereby treating the tissue defect in the subject.

Further provided is an aqueous hydrogel-forming composition for producing a macroporous hydrogel sponge of the invention, the composition comprising a mixture of: (i) a synthetic polymer or a synthetic polymer- polypeptide conjugate; (ii) a crosslinking agent; and (iii) a salt porogen; wherein the hydrogel forming-composition comprises at least 30 % by weight of said crosslinking agent.

The present invention relates further to a method of producing the synthetic polymer macroporous hydrogel sponge of the invention, said method comprising the steps:
(a) reacting an aqueous solution comprising a synthetic polymer and a crosslinking agent in the presence of a salt porogen, and subjecting the mixture to crosslinking to thereby produce a hydrogel/porogen composite; and
(b) extracting said porogen from said composite by leaching in water to thereby produce the synthetic polymer hydrogel sponge that, if desired, is further converted to the dry form.

The present invention relates still further to a method of producing a synthetic polymer-polypeptide conjugate macroporous hydrogel sponge of the invention, said method comprising the steps:
(a) reacting an aqueous solution which comprising a synthetic polymer-polypeptide conjugate and a crosslinking agent in the presence of a salt porogen, and subjecting the mixture to crosslinking to thereby produce a hydrogel/porogen composite; and
(b) extracting said porogen from said composite by leaching in water to thereby produce the synthetic polymer-polypeptide conjugate macroporous hydrogel sponge that, if desired, is further to the dry form.

As used herein, the term "synthetic polymer" is used interchangeably for "synthetic functionalized polymer" and thus the terms "PEG" and "PEG-Fib" are used interchangeably for "PEG-DA" and "PEG-DA-fibrinogen", respectively.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.
Figs. 1A-D are photomicrographs showing (A,B) lyophilized PEG-Fib/PEG-DA (1:4) 1:1 hydrogels; and (C,D) lyophilized PEG-Fib at different magnifications. (The 1:4 or 1:3 ratio relates to the volume/wt ratio between the PEG-DA and the water in which it is dissolved).
Figs. 2A-H are photomicrographs of salt leached porous scaffolds PEG-Fib/ PEG-DA 1:1 (x100); PEG-DA concentration (350:1 or 450:1 molar ratio of PEG to fibrinogen), fibrinogen concentration (8 or 10 mg/ml) and salt grains size (212-600 or 600-850 µm) are indicated.
Fig. 3 is a photomicrograph showing a sponge of PEG-Fibrinogen (10 mg/ml)/PEG-DA-PEG-DA (350:1 molar ratio of PEG to fibrinogen), 212-600 µm salt grains (x500);
Figs. 4A-D are photomicrographs showing PEG-Fibrinogen (9 mg/ml) / PEG-DA (x10) sponges- A. PEG-DA (450:1 molar ratio of PEG to fibrinogen) coarse pores, B. PEG-DA (450:1 molar ratio of PEG to fibrinogen) fine pores, C. PEG-DA (350:1 molar ratio of PEG to fibrinogen) coarse pores; D. PEG-DA (350:1 molar ratio of PEG to fibrinogen) fine pores.
Fig. 5 is a bar graph illustrating water uptake by different PEG-Fibrinogen/PEG-DA hydrogels generated according to the teachings of the present invention.
Figs. 6A-D are graphs showing the tensile properties of sponges generated according to the present teachings.
Figs. 7A-C are graphs showing the effect of protein concentration on tensile properties. A - 8 mg/ml; B - 9 mg/ml; C - 10 mg/ml.
Fig. 8 is a line graph showing proteolytic degradation of a sponge generated according to the present teachings. Note the direct correlation between protein concentration and degradation.
Figs. 9A-C are photomicrographs showing cell adhesion properties of the scaffolds of the present invention. Fig. 9A -9 mg/ml, 350:1 molar ratio of PEG to fibrinogen; Fig. 9B - 9 mg/ml, 450:1 molar ratio of PEG to fibrinogen, x10; Fig. 9C - 9 mg/ml, 450:1 molar ratio of PEG to fibrinogen, x20.
Figs. 10A-B are photomicrographs showing co-culture organization of HUVEC + HFF (human foreskin fibroblast) cells on lyophilized PEG-Fib (x25) : (A) 2 days (depth - 40 µm). (B) 9 days (depth - 16 µm).
Figs. 11A-B are photomicrographs showing co-culture organization of HUVEC + HFF on lyophilized PEG-Fib/PEG-DA (450:1 molar ratio of PEG to fibrinogen) (x25) : (A) 9 days, (B) 14 days.
Figs. 12A-D are photomicrographs showing co-culture organization of HUVEC + HFF on PEG-Fib (8 mg/ml) /PEG-DA (450:1 molar ratio of PEG to fibrinogen) 1:1, small pores (type D) : (A) 1 day in culture x25; (B) 14 days x10; (C) 14 days x25 (D) 14 days x25.
Figs. 13A-D are photomicrographs showing co-culture organization of HUVEC + HFF on PEG-Fibrinogen/PEG-DA (450:1 molar ratio of PEG to fibrinogen) 1:1,8 mg/ml, big pores, x25: A,B. 1 day C. 7 days D. 14 days.
Fig. 14 is a photomicrograph showing a co-culture of HUVEC + HFF on PEG-Fib/PEG-DA (450:1 molar ratio of PEG to fibrinogen) 1:1, 9 mg/ml, small pores, after 1 day in culture (x25).
Figs. 15A-C are photomicrographs showing co-culture organization of HUVEC + HFF on PEG-Fib/PEG-DA (350:1 molar ratio of PEG to fibrinogen), 8 mg/ml, small pores, x25: (A) 1 day (B,C) 7 days.

### DETAILED DESCRIPTION OF THE INVENTION

The present invention, in some embodiments thereof, relates to hydrogel sponges and methods of generating and using same.

As used herein the term "hydrogel sponge" refers to a three dimensional (3D) network of macromolecules typically covalently or ionically linked in which water is the dispersion medium. However, in accordance with the present invention, the hydrogel sponge may be aqueous, thus including water as the dispersion medium, or may be dehydrated so as to provide a dry sponge, as further described hereinbelow.

In accordance with the present invention, two types of macroporous hydrogel sponges are provided: the first type is herein referred to as the "synthetic polymer" hydrogel sponges, and the second type is herein referred to as the "polypeptide-synthetic polymer" hydrogel sponges. In both types, the synthetic polymer is crosslinked, wherein the crosslinking is effected in the presence of at least 30% crosslinking agent, and both types are prepared by the salt leaching technique and are characterized by high porosity and pore diameter of 50-1000 µm.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Hydrogels are networks made of water-soluble natural or synthetic polymer chains which typically contain more than 80 % water. Hydrogels are of great interest as a class of materials for tissue engineering and regeneration, as they offer three dimensional (3D) scaffolds to support the growth of cultured cells. However, one of the challenges with hydrogels has been to obtain controlled pore architectures. A high porosity and an adequate pore size are necessary to facilitate cell seeding and diffusion throughout the whole scaffold structure of both cells and nutrients.

Whilst reducing certain embodiments of the present invention to practice, the present inventors have designed a polypeptide-synthetic polymer hydrogel sponge which combines the advantages of biosynthetic hydrogels and porous microstructures for better control and support of the development of microvascular network.

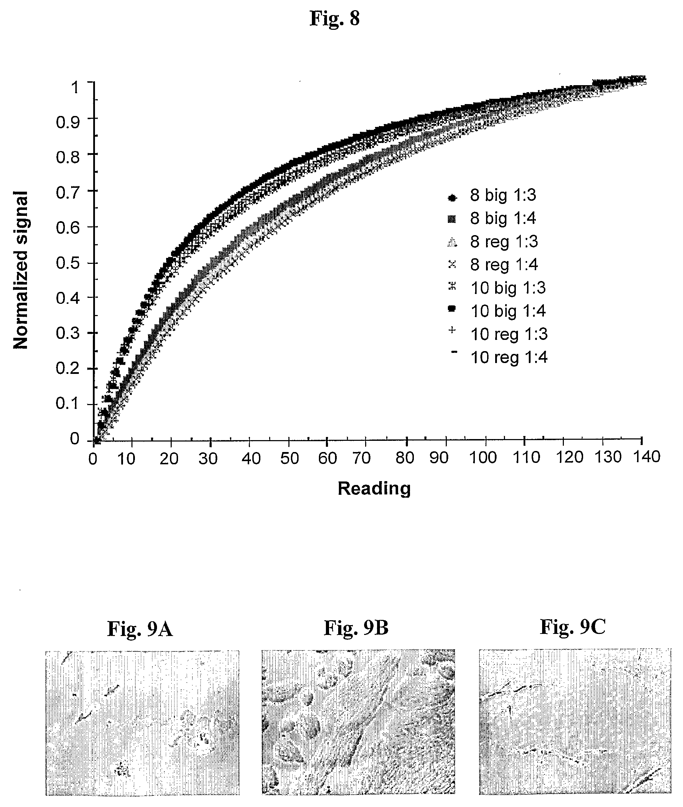

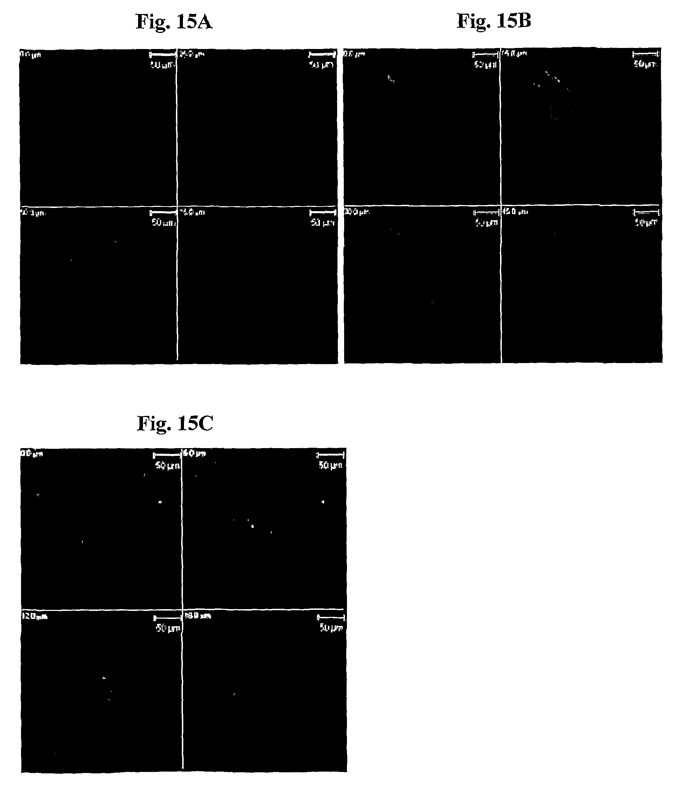

As is illustrated hereinbelow and in the Examples section which follows, the present inventors have used in certain embodiments the salt leaching method to create a variety of macroporous biosynthetic polypeptide-synthetic polymer sponge hydrogels. Specifically, the present inventors have produced a hydrogel forming aqueous solution which comprises PEG-fibrinogen and the crosslinker PEG-DA. The solution was placed in a mold comprising a salt porogen and reacted by photoinitiation to produce a hydrogel sponge being more than 70 % porous with a pore diameter 100 - 600 µm. The resultant sponge hydrogels were characterized in terms of their chemical, physical and mechanical properties. The hydrogel sponge can support the growth of a blood vasculature as evidenced by the co-culturing of human endothelial cells and fibroblasts (see Figs. 15A-C). The biocompatibility and degradability can be governed by controlling the protein concentration within the hydrogel (see Fig. 8).

The polypeptide-synthetic polymer and the 100% synthetic polymer hydrogel sponges generated according to the present invention are devoid of organic solvent and therefore are non-toxic and can be readily implemented in therapy. Furthermore, the hydrogel sponges can be presented in dry form and hydrated before application and as such can be directly injected into a defected tissue area, avoiding the need for *in-vivo* polymerization.

In the synthetic polymer-polypeptide hydrogel sponges of the invention, the polypeptides, interchangeably referred to herein as proteins, refer to polypeptides or proteins which may be synthetic, naturally occurring or denatured protein or functional fragments thereof. Such polypeptides may have a known or unknown structure, function, or molecular properties. As used herein, the term "functional fragment" of a polypeptide refers to a fragment that maintains all the desired biological and binding characteristics of the whole polypeptide.

In certain preferred embodiments, the polypeptide of the polypeptide-synthetic polymer hydrogel sponge of the invention is an extracellular matrix protein such as, but not limited to, fibrinogen, fibronectin, vimentin, microtubule-associated protein 1b, neurite outgrowth factor, bacterial cellulose, laminin or gelatin. In one more preferred embodiment, the polypeptide is fibrinogen.

The hydrogel sponge of the present invention comprises pores which according to some embodiments of the present invention generate channels.

The level of porosity and the pore diameter may be controlled during the sponge manufacturing process, as will be further described hereinbelow.

As mentioned, the polypeptide-synthetic polymer hydrogel sponge of the invention is generated using a precursor aqueous hydrogel forming composition comprising a mixture of: (i) a synthetic polymer or a polypeptide covalently attached to a synthetic polymer; (ii) a salt porogen; and (iii) a crosslinking agent; wherein the hydrogel forming composition comprises at least 30 %, 35 %, 40 %, 45 %, 50 %, 55 %, 60 %, 65 %, 70 %, 75 %, 80 %, 85 %, 90 %, 95 % by weight of the crosslinking agent. It is to be understood that when the synthetic polymer hydrogel sponge is generated and the synthetic polymer and the crosslinking agent are identical (for example, both are PEG-DA), the amount of the crosslinking agent may be below 30%.

It will be readily appreciated by the skilled artisan that the polypeptide or functional fragment thereof in the synthetic polymer-polypeptide hydrogel sponge of the invention imparts cell attachment and migration properties as well as exerts biological influences that may modify the behavior of cells. In addition, the polypeptide portion may also govern degradability of the sponge. This will very much depend on the amino acid composition (e.g., synthetic or naturally occurring amino acids as well as D or L stereochemistry), the number of synthetic polymer molecules attached to the polypeptide as well as the overall molarity of the cross-linking agent in the sponge. Thus, a higher molar ratio is expected to result in less biodegradability due to potential masking of degradation sites on the polypeptide. Those of skill in the art are capable of adjusting the molar ratio between the synthetic polymer and the protein to obtain the desired scaffold with the optimal physical and biological characteristics. A typical molar ratio between synthetic polymer and protein may be 1:1, 2:1, 10:1, 20:1, 30:1, 50:1, 100:1, 200: 1 and 300:1.

Fibrinogen and PEG are provided hereinbelow as an exemplary embodiment for such a calculation. Thus, since each fibrinogen molecule includes 29-31 potential sites which can bind to PEG, the PEG-fibrinogen component can be prepared using a wide range of molar ratios. Preferably, the molar ratio used by the present invention is 2-600 (PEG) to 1 (fibrinogen), more preferably, the molar ratio is 30-600 (PEG) to 1 (fibrinogen), more preferably, the molar ratio is 200-450 (PEG) to 1 (fibrinogen), most preferably, the molar ratio is 350-450 (PEG) to 1 (fibrinogen).

In accordance with a preferred embodiment, the polypeptide is fibrinogen. The fibrinogen used by the present invention can be whole (intact) denatured fibrinogen (*i.e.,* uncleaved chain) or fragmented fibrinogen, which can be obtained using, for example, CNBr cleavage.

Fibrinogen can be readily purified from human blood plasma using standard protein purification techniques. Purified components may be subject to antiviral treatments. Heat-treatment and solvent/detergent treatments are both commonly used in the production of fibrinogen. Fibrinogen used in accordance with the present invention is preferably pure though other components may be present. Thus products may also contain tranexamic acid, aprotinin or factor XIII. Fibrinogen is commercially available.

The synthetic polymer used in the present invention both for the polypeptide-synthetic polymer and the synthetic polymer hydrogel sponges is a functionalized polymer which is made of a synthetic material, *i.e.*, a non-natural, non-cellular material. Non-limiting examples of synthetic polymers which can be used include, without being limited to, a polyalkylene glycol, e.g., polyethylene glycol (PEG) and derivatives thereof, hydroxyapatite/polycaprolactone (HA/PLC), polyglycolic acid (PGA), poly-L-lactic acid (PLLA), polymethyl methacrylate (PMMA), polyhydroxyalkanoate (PHA), poly-4-hydroxybutyrate (P4HB), polypropylene fumarate (PPF), and polytetrafluoroethylene (PTFE).

In accordance with the present invention, a homogeneous or heterogeneous composition of synthetic polymers can be used. Each of the synthetic polymers should have at least two functional groups: at least one functional group such as sulfhydryl, carboxy or amino, which is capable of forming a direct or indirect bond with a polypeptide, for example, to a side chain thereof or to an end group of the polypeptide, or to an end group of the same or different synthetic polymer, and one or more functional groups that will copolymerize for formation of the hydrogel.

According to presently preferred embodiments of the present invention, a sole synthetic polymer is used, and such polymer is PEG, namely, functionalized PEG. The PEG molecule used by the present invention can be linear or branched, e.g., 2-arm, 4-arm, 6-arm and 8-arm PEG, and can be of any molecular weight, for example, within the range 0.4 kDa - 20 kDA, e.g., 0.4 kDa, 1.5 kDa, 4 kDa, 6 kDa, 10kDa and 20 kDa for linear or 2-arm PEG, 14 kDa or 20 kDa for 4-arm PEG, and 14 kDa and 20 kDa for 8-arm star-shaped PEG and combinations thereof.

Functionalized PEG molecules are known in the art for PEGylation of polypeptides and proteins and usually carry groups such as acrylate (Ac), vinylsulfone (VS), aldehyde, tosyl, tresyl, and the like, to mediate binding to a polypeptide. Methods of preparing functionalized PEG molecules are known in the art. For example, PEG-VS can be prepared under argon by reacting a dichloromethane (DCM) solution of the PEG-OH with NaOH and then with di-vinylsulfone (molar ratios: OH 1: NaH 5: divinyl sulfone 50, at 0.2 gram PEG/mL DCM). PEG-Ac is made under argon by reacting a DCM solution of the PEG-OH with acryloyl chloride and triethylamine (molar ratios: OH 1: acryloyl chloride 1.5: triethylamine 2, at 0.2 gram PEG/mL DCM). Another example of functionalized PEG is polyethylene glycol-dimethacrylate (PEG-DMA),

It will be appreciated that such chemical groups can be attached to linear, 2-arm, 4-arm, 6-arm or 8-arm PEG molecules. Thus, according to some embodiments, the PEG-Ac used by the present invention is PEG-diacrylate (PEG-DA) or 4-arm, 6-arm or 8-arm star-shaped PEG multiacrylate.

As used herein the term "crosslinking agent" relates to a multifunctional, such as difunctional, substance that promotes covalent bonding between polymer chains by reacting with reactive groups of the polymer to thereby lead to crosslinking.

In preferred embodiments, the crosslinking agent is a synthetic polymer such as, but not limited to, PEG-DA, PEG multi-acrylate, and/or PEG-VS or PEG-Bis- di(VS). In some preferred embodiments, the crosslinking agent is identical to the synthetic polymer and is, for example, PEG-DA.

The concentration of the crosslinking molecules (e.g., PEG-DA) can affect the sponge's strength, flexibility, elasticity and biodegradability and determination of such a concentration depends on the scaffold application and is within the capabilities of those skilled in the arts. For example, the higher the concentration of the crosslinking agent the higher the rigidity and poorer the biodegradability and biocompatibility.

As mentioned, the present invention also provides hydrogel sponges containing no polypeptide and consisting of 100% crosslinked synthetic polymer being at least 20 %, 30 %, 40 %, 50 %, 60 %, 65 %, 70 %, 75 %, 80 %, 85 %, 90 % or more porous and having a pore diameter of 50-1000 µm; alternatively about 100 to about 600 µm; alternatively about 100 to about 400 µm; and alternatively or additionally about 400 to about 600 µm.

It will be evident that such a 100% crosslinked synthetic polymer sponge does not support cell growth *per se* but is highly stable and can be effectively used as a blood vessel blocking agent such as for prevention of tumor growth as will be further described hereinbelow. Since this sponge is not biodegradable, it can be used as a long term implant. In addition, if such sponges are coated with cell adhesion promoters, they can be used also to support cell growth and may be then used for tissue regeneration.

As used herein, the term "polyalkylene glycol" encompasses any compound that comprises a chain of alkylene radicals interrupted by oxygen atoms. This term, however, further encompasses, according to the present embodiments, analogs of polyalkylene glycols in which one or more of the oxygen atoms are replaced by another heteroatom such as S and/or N. The polyalkylene glycols described herein can therefore be comprised, for example, of fragments of aminoalkylenes, oxyalkylenes, and/or thiolkylenes, wherein each of the alkylene groups can be further substituted. If the polyakylene glycol is branched, the chains can be the same or different, in terms of the length of the alkylene chain, the substituents and/or the heteroatom, and are preferably the same.

Thus, according to preferred embodiments of the present invention, the polyalkylene glycol moiety is represented by the general Formula I:

-[U(CR'R")m]nV Formula I

wherein:
m is an integer from 1 to 6, representing the length of the alkylene chain in each of the fragments composing the polyalkylene glycol moiety;
n is an integer from 100 to 100,000 representing the number of fragments composing the polyalkylene glycol moiety;
U is O, S or NR"', representing the heteroatom interrupting the alkylene chain in each fragment;
V is a functional group, representing the end group(s) in the polyalkylene glycol moiety, as is detailed hereinbelow; and
R', R" and R"' representing the substituents of the polyalkylene glycol moiety, are each independently selected from hydrogen, alkyl, aryl and cycloalkyl.

Preferably, the polyalkylene glycol moiety is derived from polyalkylene glycol and hence U in Formula I above is O. The functional group V is preferably a group containing a double bond such as acrylate, methacrylate and vinyl sulfone groups and is present at the two terminal ends of the polyalkylene glycol, if it is linear, or at more than two terminal ends, if the polymer is branched. Substituted and non-substituted PEGs, having various chain length (in terms of the number of fragments, n) are commercially available compounds, which are typically known as pharmaceutically acceptable compounds and hence are highly advantageous for use in the context of the present embodiments. In preferred embodiments, the polyalkylene glycol is polyethylene glycol (PEG) (m in Formula I above equals 2), which is functionalized by acrylate groups at the two end groups in linear PEG, namely PEG-DA, or in more than 2 end groups in branched PEG, namely, PEG-multiacrylate.

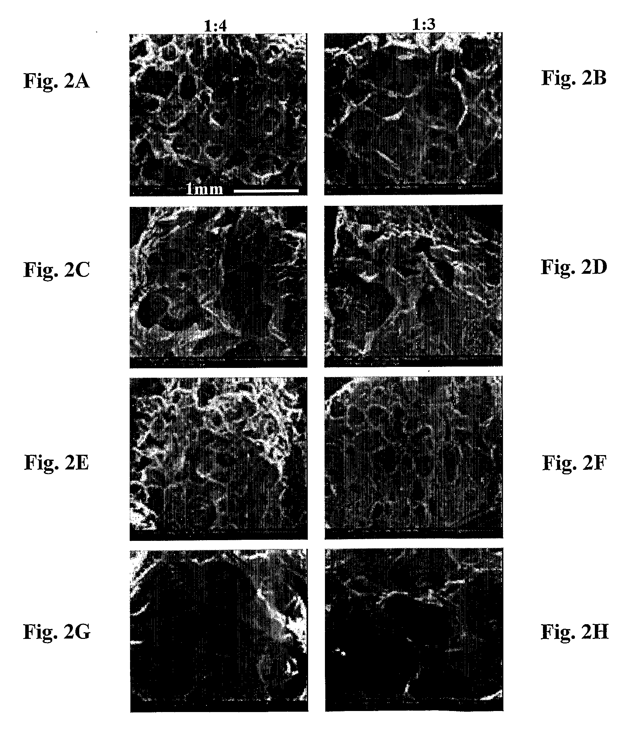

In accordance with the present invention, both types of hydrogel sponges of the present invention are generated using the salt leaching method. This method allows the preparation of porous scaffolds having regular porosity as shown for salt leached PEG-fibrinogen crosslinked with PEG-DA (denoted herein as PEG-Fib/PEG-DA) in Figs. 2A-H.

The salt leaching method is conducted by reacting the hydrogel forming composition comprising the polypeptide attached to uncrosslinked synthetic polymer (for the polypeptide-synthetic polymer hydrogel sponges) or the synthetic polymer alone (for the 100 % synthetic polymer hydrogel sponges) and the crosslinking agent in the presence of a salt porogen, subjecting the mixture to crosslinking to thereby produce a hydrogel/porogen composite; and extracting the porogen from the composite to thereby produce the desired macroporous hydrogel sponge.

Thus, the aqueous hydrogel forming solution is cast into a mold designed according to the intended use and filled with porogen particles. Examples of effective porogens are sodium chloride or other easily water dissolved salts, sucrose, lactose, and dextrin. In a preferred embodiment, the porogen is sodium chloride. The size of the porogen particles will affect the size (diameter) of the sponge pores, while the polymer to porogen ratio is directly correlated to the amount of porosity of the final structure. The solution is allowed to polymerize/crosslink using any method known in the art.

Thus, reacting can be effected by a free-radical polymerization reaction (*i.e.,* a crosslinking reaction). For example, crosslinking via photoinitiation in the presence of light of an appropriate wavelength, e.g., 365 nm; chemical crosslinking in the presence of a free-radical donor; and/or heating at the appropriate temperatures. According to an exemplary embodiment, crosslinking is effected by photoinitiation.

Photoinitiation can take place using a photoinitiation agent (*i.e*., photoinitiator) such as bis(2,4,6-trimethylbenzoyl) phenylphosphine oxide (BAPO), 2,2-dimethoxy-2-phenylacetophenone (DMPA), camphorquinone (CQ), 1-phenyl-1,2-propanedione (PPD), the organometallic complex Cp'Pt(CH(3))(3) (Cp' = eta(5)-C(5)H(4)CH(3)), 2-hydroxy-1-[4-(hydroxyethoxy)phenyl]-2-methyl-l-propanone (Irgacure® 2959), dimethylaminoethyl methacrylate (DMAEMA), 2,2-dimethoxy-2-phenylacetophenone, benzophenone (BP), or flavin.

The photoinitiation reaction can be performed using a variety of wavelengths including UV (190-365 nm) wavelengths and visible light (400-1100 nm) and at various light intensities. It will be appreciated that for therapeutic applications the photoinitiator is preferably non-toxic, non-hazardous.

The hydrogel/porogen composite structure thus obtained is immersed in a water bath for dissolving the porogen, and then washed so as to completely remove/extract the porogen (e.g., three exchanges each hour for 5 hours to assure complete removal). Once the porogen has been fully dissolved, a porous structure is obtained. The sponge is then subjected to lyophilization such as by freeze-drying, so as to obtain a dry sponge which may then be hydrated so as to render it injectable. Sponges may be sterilized in the dry or wet form by autoclaving or treatment with ethylene oxide gas, UV irradiation or alcohol.

This salt leaching method yields sponges having high porosity and high compressive strength.

Thus according to an exemplary embodiment, the sponge has a porosity of at least, 60 %, 70 %, 80 %, 90 %, 95 % and, more preferably, about 80-95 %. The pores are preferably homogenous and interconnected. As mentioned, pore size in the sponge is determined by the size of the salt particles used in the salt leaching process. Larger salt particles (e.g., coarse 600-850 µm) yield larger pores in the sponge. Fine particles (e.g., fine 212-600 µm or 100 µm) will provide for smaller pores. A homogeneous or heterogeneous composition of salt particles may be used based on the end use of the sponge. According to an exemplary embodiment, at least 5 % (vol), 10 % (vol), 20 % (vol), 30 % (vol), 40 % (vol), 50 % (vol), 60 % (vol), 70 % (vol), 80 % (vol), 90 % (vol) of the pores have a pore diameter of about 50 to about 1200 µm, alternatively about 50 to about 1000 µm, alternatively about 100 to about 600 µm; alternatively about 100 to about 400 µm; and alternatively or additionally about 400 to about 600 µm. The preferred diameter depends on the cell type or required mechanical properties/function, and also on the composition. For compositions of higher polymer content or higher stiffness, usually larger pores, >250 µm are preferred.

According to exemplary embodiments the sponge of the present invention has a tensile strength of at least about 0.2 KPa, 1 KPa, at the range of 1-20 kPa, 10-50 kPa, 70-100 kPa, or 0.2-200 kPa, and a range of strain to failure of 10-200 %, 75-95 %, 50-100 % or 30-150 %.

Both types of sponges of the instant invention may be modified with one or more molecules that may be attached to, coated on, embedded or impregnated in the sponge. Inclusion of such molecules may increase the biodegradability and/or biofunctionality of the hydrogel of the present invention, as needed.

Thus, for example, hydrogels of the present invention may enclose nonreactive components (namely, nonreactive with the hydrogel). Examples of such nonreactive components may include one or more non-pharmaceutical agents or pharmaceutical agents selected from disinfectants, chemotherapeutics, antiangiogenic agents, antimicrobial agents, antiviral agents, hemostatics, antiphlogistics, anesthetics, analgesics, nutritional supplements, or biopolymers such as polypeptides, proteins, plasma derivatives (proteins such as immune globulin, plasma protein fraction, albumin, antihemophilic factor), enzymes or mixtures thereof. In other words, nonreactive components may be combined with the hydrogel to provide stabilization or protection of these components. Such combined composition may be prepared, for example, by dissolving or suspending the nonreactive components in the aqueous medium to be used for gelation before effecting the gelation (namely, before effecting the crosslinking in aqueous environment). Methods of loading hydrogels with pharmaceuticals are well known in the art [see for example, drug inclusion in bovine serum albumin hydrogels described in Gayet and Fortier (1995) Art. Cells. Blood Subs. And Immob. Biotech. 23(5), 605-611].

Alternatively or additionally, the biodegradability and biofunctionality of the hydrogel sponge of the present invention can be further increased by attaching to or impregnating the sponge with a protein such as a cell signaling protein or a growth factor. Attaching such proteins to the hydrogel of the present invention is preferably employed by covalent immobilization of a PEGylated protein to the PEG hydrogel network during cross-linking (Seliktar et al 2004). The immobilization of such factor is accomplished by directly reacting functionalized PEG to an unreacted sulfhydryl group present on a cysteine residue of the protein sequence. Impregnation of the hydrogel with growth factors can be performed by dehydrating the sponge and then immersing the hydrogels in a solution containing the growth factors and gently shaking such hydrogels for a few hours until the growth factors penetrate the sponge during the hydration process. Likewise, the hydrogel can be impregnated with growth factors by incubation in growth factor-containing solution overnight until the growth factor diffuses into the polymeric network of the sponge by slow, passive diffusion. The latter is influenced by the degree of cross-linking, the porosity of the sponge, and the structural properties described hereinabove.

Exemplary agents that may be incorporated into the sponge of the present invention include, but are not limited to those that promote cell adhesion (e.g. fibronectin, integrins), cell colonization, cell proliferation, cell differentiation, cell extravasation and/or cell migration. Thus, for example, the agent may be an amino acid, a small molecule chemical, a peptide, a polypeptide, a protein, a DNA, an RNA, a lipid and/or a proteoglycan.

Proteins that may be incorporated into the sponges of the present invention include, but are not limited to, extracellular matrix proteins, cell adhesion proteins, growth factors, cytokines, hormones, proteases and protease substrates. Thus, exemplary proteins include vascular endothelial-derived growth factor (VEGF), activin-A, EGF, bone morphogenetic protein (BMF), TGFβ, HGF, PDGF, TGFα, IGF-I and II, hematopoietic growth factors, heparin binding growth factor, peptide growth factors, EPO, interleukins (ILs), tumor necrosis factors (TNFs), interferons (IFNs), colony stimulating factors, FGF-A and B, NGF or muscle morphogenic factor (MMP). The particular growth factor employed should be appropriate to the desired cell activity. The regulatory effects of a large family of growth factors are well known to those skilled in the art.

The hydrogel sponges of the present invention comprising the polypeptide attached to the synthetic polymer can be used to support cell growth, attachment and spreading, and thus facilitate cell growth, tissue regeneration and/or tissue repair. Since the hydrogel sponge is already fully cross-linked at its gel state it can be easily placed into a target tissue region or gaps within a tissue or an organ with a simple injection, following which it can fill the void and/or initiate the process of regeneration as the sponge degrades away. The injection of the fully crosslinked product avoids the application of radiation to the treated tissue as known in the art for UV-cured hydrogels applied to tissues before crosslinking. Moreover, it avoids the need for surgical interruption, where the needed void fill and/or regeneration location are easily approached from outside.

The hydrogel sponge can be designed in the required shape and size before its application into the body and its location can be very well specified and controlled.

The cross-linked hydrogel sponges of the invention can be used, for example, for cosmetic repair, and as a scaffold for tissue culture and tissue engineering.

By changing the proportions between the synthetic polymer (used also as crosslinker) and the polypeptide, it is possible to tailor the mechanical properties as well as the degradation rate. This ability is very important and enables tuning of the product according to the application needs. Hydrogels comprising 50 % vol PEG-DA will degrade completely after 3-4 years. Theoretically, 100 % PEG-DA will "never" degrade. 100% PEG-fibrinogen degrades after 2 weeks.

The salt leaching method is carried out only in water such that the hydrogel sponge end product has no undesired residues of organic solvents or dangerous chemicals.

The hydrogel sponge of the present invention can be supplied and stored in its dry state (after lyophilization as mentioned before, and sterilization, if desired). The dry product has a long shelf life. Before actual application either to the body or in a laboratory experiment, it can be wetted thus turning to its gel state.

Thus, in certain embodiments, the present invention provides a method of treating a tissue defect in a subject in need thereof, the method comprising administering to the subject a polypeptide-synthetic polymer or a 100% synthetic polymer hydrogel sponge of the present invention to thereby treat the tissue defect. It will be understood that when a synthetic polymer hydrogel sponge is used for tissue regeneration, it is coated with agents that promote cell adhesion and agents that support cell growth and proliferation.

The phrase "tissue" refers to part of an organism consisting of an aggregate of cells having a similar structure and function. Examples include, but are not limited to, brain tissue, retina, skin tissue, hepatic tissue, pancreatic tissue, bone, nerve, cartilage, connective tissue, muscle tissue, cardiac tissue brain tissue, vascular tissue, renal tissue, pulmonary tissue, gonadal tissue and fat tissue. In exemplary embodiments, the phrase "tissue" as used herein also encompasses the phrase "organ" which refers to a fully differentiated structural and functional unit in an animal that is specialized for some particular function. Non-limiting examples of organs include breast, skin, head, brain, eye, leg, hand, heart, liver kidney, lung, pancreas, ovary, testis, and stomach.

"Tissue defect", "tissue damage" or "tissue loss" refers to any disorder, disease or condition exhibiting a tissue damage (i.e., non-functioning tissue, cancerous or pre-cancerous tissue, broken tissue, fractured tissue, fibrotic tissue, or ischemic tissue) or a tissue loss (e.g., following a trauma, an infectious disease, a genetic disease, and the like) which require tissue regeneration. The condition may be pathogenic or cosmetic (e.g., skin wrinkling). Examples of conditions requiring tissue regeneration include, but are not limited to, skin damage related to, for example, aging (wrinkles), wounds, burns, ulcers, liver cirrhosis such as in hepatitis C patients (liver), Type²-1 diabetes (pancreas), cystic fibrosis (lung, liver, pancreas), bone cancer (bone), age-related macular degeneration (retina), myocardial infarction, myocardial repair, CNS lesions (myelin), articular cartilage defects (chondrocytes), bladder degeneration, intestinal degeneration, and the like.

The phrase "treating" refers to inhibiting or arresting the development of a disease, disorder or condition and/or causing the reduction, remission, or regression of the condition in an individual suffering from, or diagnosed with, the condition. Those of skill in the art will be aware of various methodologies and assays which can be used to assess the development of a disease, disorder or condition, and similarly, various methodologies and assays which can be used to assess the reduction, remission or regression of a disease, disorder or condition.

The method is effected by administering (i.e., injecting or implanting) of the present invention alone or following *ex-vivo* seeding with cells, into the affected tissue of the subject to thereby induce formation of the tissue and treat the tissue defect.

It will be appreciated that the cells seeded on the polypeptide-synthetic polymer sponge for *ex-vivo* formation of a tissue can be derived from the treated individual (autologous source) or from allogeneic sources such as embryonic stem cells which are not expected to induce an immunogenic reaction.

In certain embodiments, following *ex-vivo* tissue formation, the seeded polypeptide-synthetic polymer sponge is implanted in the subject. Those of skill in the art are capable of determining when and how to implant the sponge to thereby induce tissue regeneration.

The polypeptide-synthetic polymer sponge *per se* or loaded with cells (selected according to the intended use) are preferably administered to the subject by way of injection into the affected tissue region. In case a dry sponge is used it can be implanted in the subject using a surgical tool such as a scalpel, spoon, spatula, or other surgical device.

The present invention further contemplates blocking blood vessels using a synthetic polymer hydrogel sponge of the invention, which does not support tissue regeneration. The polymer is preferably PEG-DA. The sponge can be used, for example, for blocking blood vessels of tumors, thus abrogating tumor growth. It is appreciated that in order to survive and grow, tumors need to produce new blood vessels so that they can obtain blood and oxygen. In this case, the hydrogel sponge is intratumorally injected into the blood vessel. Since the injected material is already *ex-vivo* crosslinked it is expected to remain in the target tissue and not affect healthy tissues. In such a configuration the sponge may be loaded with chemotherapeutic agents and/or anti-angiogenic agents (e.g., avastin). Such a treatment may also be effective in the treatment of hypervasculature such as in the condition of spider veins/varicose veins or aneurysm.

The 100 % synthetic polymer hydrogel sponges of the invention can be used for any purpose that do not require tissue regeneration or sustainability for a very long time. Thus, besides blocking blood vessels, they can be used for filling a void in implants and as flexible stents.

Compositions of the present invention may, if desired, be presented in a pack or dispenser device, such as an FDA-approved kit, which may contain one or more unit dosage forms such as for personalized use containing the active ingredient and optionally sterile disposable means for delivery (e.g., syringe). The pack may, for example, comprise metal or plastic foil, such as a blister pack. The pack or dispenser device may be accompanied by instructions for administration. The pack or dispenser device may also be accompanied by a notice in a form prescribed by a governmental agency regulating the manufacture, use, or sale of pharmaceuticals, which notice is reflective of approval by the agency of the form of the compositions for human or veterinary administration. Such notice, for example, may include labeling approved by the U.S. Food and Drug Administration for prescription drugs or of an approved product insert. Compositions comprising a preparation of the invention formulated in a pharmaceutically acceptable carrier may also be prepared, placed in an appropriate container, and labeled for treatment of an indicated condition, as further detailed above.

As used herein the term "about" refers to ± 10 %.

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

Various embodiments and aspects of the present invention as delineated hereinabove and as claimed in the claims section below find experimental support in the following examples.

### EXAMPLES

### Experimental Procedures

*(**i**) **Scaffold Fabrication*** - Lyophilized sponge-like PEG-Fib based gels with or without additional PEG-DA were produced by freezing in (-80°C) for 4 hours and lyophilization over night. The hydrogel with PEG -DA was prepared by adding PEG-DA/ PEG-fibrinogen to sodium chloride porogen and crosslinking using UV irradiation (λ=365 nm). After salt leaching in water, the resulting sponge hydrogel was freezed in (-80°C) for 4 hours and lyophilized over night.
***(ii)*** ***Synthesis of PEG Diacrylate*** (***PEG***-***DA***) - PEG-diacrylate (PEG-DA) was prepared from linear PEG, MW = 10-kDa (Fluka, Buchs, Switzerland), essentially as described elsewhere (Lutolf and Hubbell, Biomacromolecules, 2003, 713-722; Elbert D.L., et al., J. Control Release, 2001, 11-25). Briefly, acrylation of PEG-OH was carried out under Argon by reacting a dichloromethane (DCM) (Aldrich, Sleeze, Germany) solution of the PEG-OH with acryloyl chloride (Merck, Darmstadt, Germany) and triethylamine (Fluka) at a molar ratio of 1-PEG-OH to 1.5 acryloyl chloride to 1.5 triethylamine (0.2 g PEG/ml DCM). The final product was precipitated in the presence of ice-cold diethyl ether and dried under vacuum overnight. The degree of end-group conversion was confirmed by ¹H NMR and was found to be 97-99 % (data not shown).
*(**iii**)* ***Cyanogen bromide cleavage of fibrinogen*** - Whole fibrinogen (Fib) (Sigma-Aldrich, Steinheim, Germany, Cat # F8630) was dissolved in a solution of 70% formic acid containing 17 mg/ml cyanogen bromide (CNBr) (Aldrich, Cat. # C9, 149-2) and incubated overnight in the dark at 25 °C. The cleaved fibrinogen fragments were dialyzed for 2 days at 4 °C in 50 mM phosphate buffered saline (PBS) at pH 7.4 with a twice-daily change of buffer to remove all the CNBr and formic acid from the solution. The dialyzed fragments were stored in PBS at 4 °C before PEGylation. Since grafting of PEG has a detectable effect on the mobility of the protein in acrylamide gels (Kurfurst MM, Anal. Biochem., 1992, 244-248; Pomroy NC and Deber CM, Biochem Biophys Res Commun, 1998; 618-21), visualization of the PEGylated fibrinogen fragments was performed using SDS-PAGE followed by a Coomassie®-blue staining.
(***iv***) ***Microstructure characterization** -* The microstructure of the dry scaffolds was characterized by scanning electron microscope (SEM) (Quanta 200, FEI, Netherlands). The scaffold samples were gold coated using Polaron gold coater operated at 0.1 Torr 20 mA, and were observed under vacuum of 1.7 x 10⁻⁶ Torr, 10-20 kV and emission current of 100 µA. The microstructure of the wet scaffolds was characterized by confocal microscope (LSM 510, Zeiss, Germany), using N-hydroxysuccinimide(NHS)-fluorescein (Pierce), a fluorescent marker which efficiently binds proteins.
***(v) Swell test*** - Hydrogels were tested for swelling by immersion of lyophilized sponges in phosphate buffer solution (PBS) for 2 hours, and weighing the wet weight compared with the initial dry one.

### (vi) Mechanical properties testing

***Oscillating stress** -* The mechanical properties of the sponge hydrogels were evaluated under oscilating strain using TA Instruments AR-G2 rheometer. Based on definition of the linear viscoelastic region, a constant strain of 1 % was set, and the hydrogels were tested for a frequency range of 0.01 - 10 Hz. At the said chosen strain, only the range of 0.01 - 0.5 Hz was found to be linear.

***Tensile test*** - Tensile properties of the porous hydrogels were evaluated for wet scaffolds (~7 x 15 x 1 mm³) under constant strain rate of 0.1 mm/s until failure was reached, using an Instron™ 5544 apparatus. The samples edges were glued to a plastic foil rim for mechanical support when attached to the tensile machine grips.
***(vii) Cell culture** -* HFF (human foreskin fibroblasts) were cultured in Dulbecco's modified Eagle medium (DMEM) (Invitrogen) supplemented with 10% fetal bovine serum (FBS) (Hyclone), 1% L-glutamine (Gibco) and 0.2 % β-mercapto-ethanol. Human umbilical vein endothelial cells (HUVEC) (Clonetics) were cultured in endothelial growth medium 2 (EGM-2) (Clonetics). The hydrogel sponges were cut into ≈ 3 x 5 x 1 mm³, and were sterilized by UV at λ=365 nm for 30 minutes. 1.2 x 10⁶ HUVEC cells/ml and 0.2 x 10⁶ HFF cells/ml were suspended in 12 µl medium and seeded onto the sponges. The hydrogel sponges swelled immediately after wetting with the cell suspension. Following 30 minutes of incubation at 37 °C allowing cell adhesion to scaffold, culture medium was added, and the samples were further cultured at 37 °C. The culture medium was replaced 3 times a week.
(***viii***) ***Adhesion test*** - PEG-Fib/PEG-DA (about 350:1 or about 450:1 molar ratio of PEG to fibrinogen) compositions, consisting of about 9 mg/ml protein and two different PEG-DA concentrations in PBS, were cast and cured into disks ~ 1mm thick x 10 mm in diameter, having smooth surface. The gel disks were left to swell overnight in 12-well dishes suspended in culture medium at 37°C. 100,000 HFF and HUVEC cells in ratio 3:7, respectively, suspended in 200 µl culture medium, were added to each disk. The cells were allowed to settle on the disks for 30 min, and additional 1 ml culture medium was poured to each well. The culture medium was replaced every 2 days and the disks were taken out of the wells after 5 days in culture. Viability and proliferation of the cells on each gel type were qualitatively evaluated using an optical microscope (Nikon TS100).
***(ix) Proteolytic digestion** -* Hydrogel sponges of several compositions and different pore sizes (Table 1, above) were labeled with NHS-fluorescein (Pierce). NHS-fluorescein is an N-hydroxysuccinimide (NHS)-ester that reacts efficiently with primary amino groups, therefore, is commonly used for labeling proteins. It binds selectively the fibrinogen backbone of the hydrogel. The degradation rate was characterized in 0.5 mg/ml trypsin solution (Sigma Aldrich) in 150 mM PBS (pH 7.4) containing 0.1 % sodium azide. The fluorescently labeled fibrinogen (excitation at λ=491 nm, and emission at λ=518 nm) that was released from the sponge hydrogel, was measured using a UV spectrophotometer. The unreacted fluorescein was removed by sequential washes.
***(x) Immunofluorescent staining and characterization** -* Scaffolds and cells seeded thereon were fixed in 4 % formalin and immunostained using smooth muscle actin (SMA) + Cy3 (for detection of HFF that differentiated into smooth muscle cells (SMC)) and Von Willebrand Factor (VWF) + Alexa 488 (for detection of HUVEC). 4',6-diamidino-2-phenylindole (DAPI) was used for nuclear staining. 3D characterization of the immunofluorescent stained tissue constructs was conducted using confocal microscopy.

**Example 1. Preparation of fibrinogen-PEG-DA** - To PEGylate the fibrinogen protein, tris (2-carboxyethyl) phosphine hydrochloride (TCEP·HCl) (Sigma) was added to a 7 mg/ml solution of fibrinogen in 50 mM PBS with 8 M urea (molar ratio 68:1 TCEP to fibrinogen cysteines) and the solution was left shaking for 15 min at 25 °C until fully dissolved. After dissolution of the fibrinogen, a solution of PEG-DA (250-300 mg/ml) in 50 mM PBS and 8 M urea was added to the fibrinogen and reacted overnight in the dark at 25 °C. The molar ratio of PEG to fibrinogen was 145:1 (linear PEG-DA, MW 4-kDa, 6-kDa, and 20-kDa). The final PEGylated protein product was precipitated for 20 minutes at room temperature while stirring in 5X excess acetone (Frutarom, Haifa, Israel). The precipitated PEGylated protein solution was centrifuged for 20 minutes at 5000 RPM (Sorvall GSA rotor) and the pellet was redissolved to 20 mg/ml protein concentration in PBS containing 8 M urea. The PEGylated protein solution was then dialyzed for 2 days at 4 °C against PBS containing 0.1 % (v/v) glacial acetic acid (Frutarom) with twice-daily changes of PBS (Spectrum, 12-14-kDa MW cutoff). The dialyzed product was either used immediately or lyophilized in a solution of 10 % D-(+)-glucose (Riedel-deHaen, Germany) to improve solubility upon redissolution. The lyophilized PEGylated protein was stored under Argon at -80 °C for up to six months.

### Example 2. Crosslinking and salt leaching of PEG-Fib/PEG-DA

The PEG-fibrinogen hydrogels are made of a precursor solution of PEGylated fibrinogen (whole or cleaved). The precursor solution is made by solubilizing PEGylated fibrinogen in 1-ml of 50 mM PBS, pH=7.4 and 25 °C. The precursor solution also contains a PEG-DA crosslinking constituent at a molar ratio of 1:2 PEG-DA to functional groups on the PEGylated fibrinogen. The additional PEG-DA is used to efficiently crosslink the PEGylated protein macromeres and minimize steric hindrances that cause poor gelation (Almany and Seliktar, Biomaterials, 2005, 2467-2477).

Macroporous sponge PEG-Fib/PEG-DA (in PBS) about 350:1 or about 450:1 molar ratio of PEG to fibrinogen, was produced using the salt leaching method, as follows: 100 µl of the precursor solution (the compositions are detailed in Table 1) containing 1 µl of Igracuret™2959 photoinitiator solution (Ciba Specialty Chemicals, Tarrytown, New York) in 70% ethanol (100 mg/ml) were added to 0.4 g NaCl, 212-600 (fine) or 600-850 µm (coarse) followed by UV (λ=365 nm) crosslinking for 5 minutes. The crosslinked polymerized structure was immersed in distilled water for about 6 hours; frozen over night (-80°C) and lyophilized over night. Overall, 12 different sponge hydrogels were created (Table 1, below).

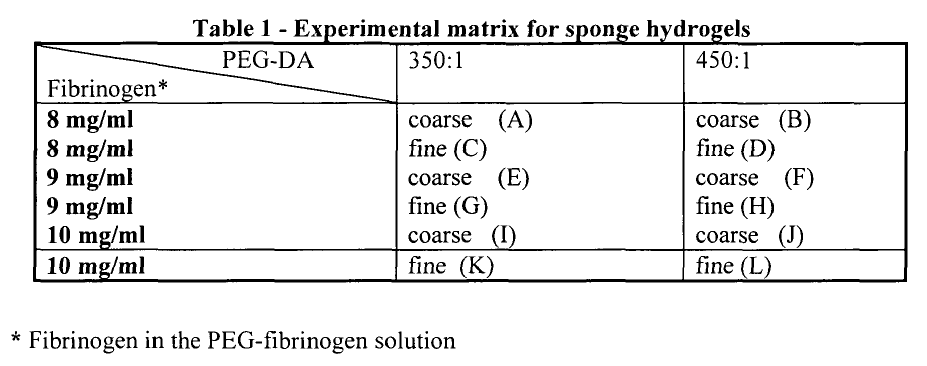

### Example 3. Scaffold microstructure of the hydrogel sponges

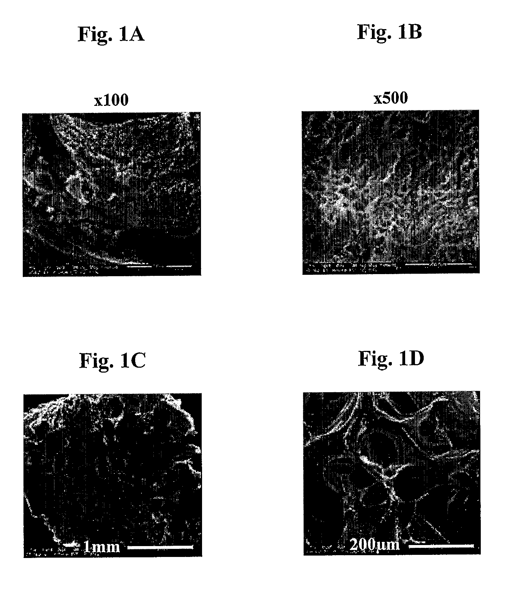

The dry scaffolds, both the lyophilized (no salt leaching) of PEG-Fib and the dry salt-leached scaffold of PEG-Fib/PEG-DA were analyzed by SEM. The resulting structure of PEG-Fib/PEG-DA1:1 sponge from the lyophilization process was nonhomogenous, and the pores were 10-20 µm in diameter (Figs. 1A-B). The lyophilized PEG-Fib seemed homogenous and its pores were roughly 100 µm in diameter (Figs. 1C,D). Yet, it was very difficult to handle since it crumbled easily. The pore size of the lyophilized samples reflects the crosslinking density of each.

The hydrogel scaffolds resulting from the salt leaching process followed by lyophilization, consisted of highly macroporous structures similar to poly(lactic-*co-*glycolic acid)/poly-L-lactic acid (PLGA/PLLA) scaffolds (Zoldan et al., 2006)

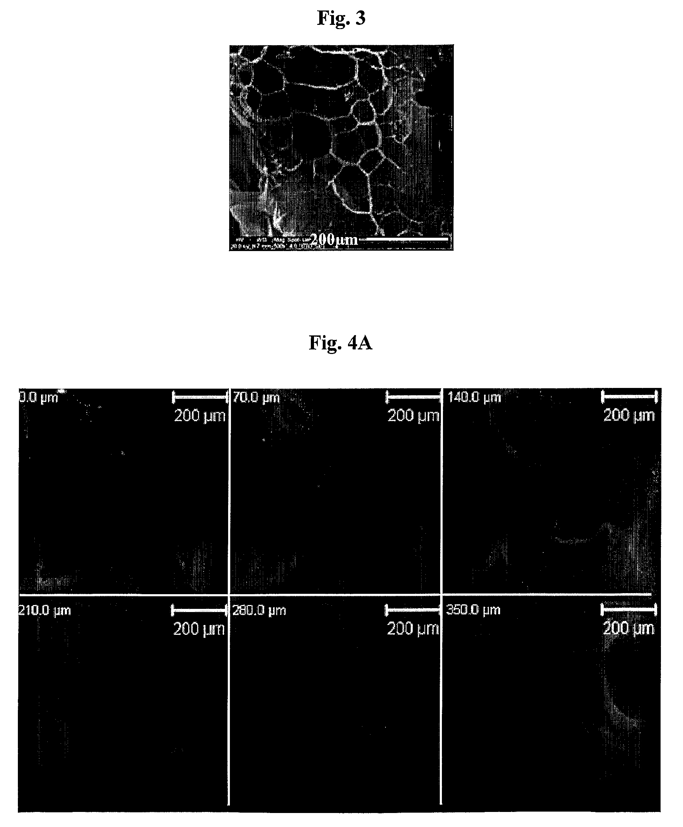

Qualitatively, one can notice a two hierarchical microstructure in the hydrogel sponges (Figs. 2A-D). Notably, the porous structure comprises 2 types of pores - tens microns in size and hundreds microns in size. The fine pores result from the mesh structure of the crosslinked gel, and the bigger ones result from washing away the salt grains. This hierarchical structure was most clearly observed in the scaffold made from PEG-Fib of highest protein concentration, 10 mg/ml, that was mixed with PEG-DA solution of 1 g in 3 ml PBS (Fig. 2H). It can be assumed that the larger pores result from the leached salt, while the smaller ones reflect the polymer network which restored water prior to lyophilization. The larger pores resulting from bigger salt grains are easily recognized as is evident from Figs. 2C,D,G,H. Easier to notice in the smaller pore scaffolds, that the increased concentration of PEG-DA, (350:1 compared to 450:1 molar ratio of PEG to fibrinogen), seemed to stabilize mechanically better the pores walls (Figs. 2B,F). Another interesting feature of the hydrogel sponges microstructure are the microfibrils seen in the scaffolds made from PEG-Fib of 10 mg/ml in protein concentration (Fig. 3).

Already during the cell seeding procedure the dry, salt leached, hydrogel sponges adsorb the cell suspension (cells in culturing medium used at the seeding stage) and swell to their equilibrium volume. Therefore, it is essential to characterize the morphology of the wet sponge hydrogels as well. As described above, the architecture of the wet sponge hydrogels was characterized using a confocal microscope and a fluorescent marker, fluorescein.

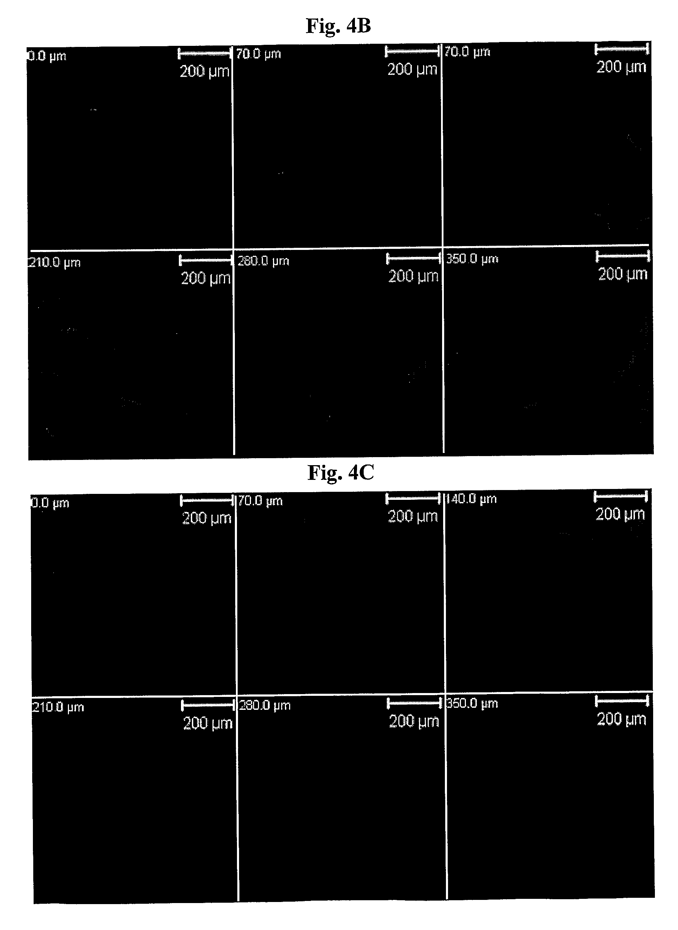

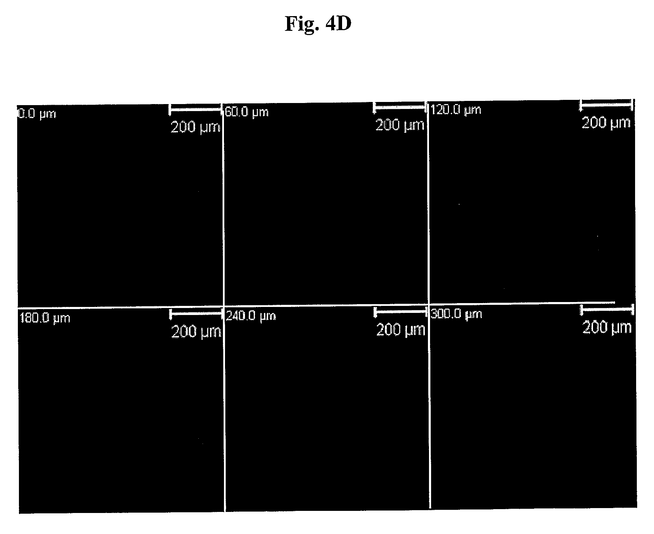

The optical images of the wet sponge hydrogels (Figs. 4A-D) revealed irregular pore geometry in accordance with the dry sponges, suggesting that the adsorbed water do not affect the pore shape. It is readily seen that pores resulting from coarse salt grains are bigger than the ones from fine salt (Figs. 4A,C *vs.* 3B,D). Most of the pores in the coarse grains sponges were ~400-600 µm, while the ones of the finer pores sponges were ~100-400 µm. Moreover, the pore walls of the 350:1 molar ratio of PEG to fibrinogen composition (Figs. 4C and 4D) seemed to be firmer than those of the 450:1. The optical images enabled rough evaluation of the pore walls thickness. In any of the sponges, the pore wall thickness is in the few tens of microns scale, as the wall thickness of the coarse pores is ~50 µm, and the one of the fine pores is ~20 µm. The difference in wall thickness is expected; the surface area of fine particles is higher than the surface area of coarse particles, therefore, the coating thickness resulting from identical amount of matrix material is lower.

### Example 4. Water uptake (swelling) properties of hydrogel sponges

The porous PEG-Fib/PEG-DA hydrogels were tested for swelling by immersion of dry hydrogel sponges in phosphate buffer solution (PBS) for 2 hours, and weighing the wet weight compared with the initial dry weight.

Both the protein and the crosslinker (PEG-DA) concentrations are expected to affect liquid absorbance capability of the gel, since they affect its mesh size and geometry. On top of that, the macropores created by the leached salt can serve as free space for holding extra liquid. The overall water uptake capability of the gels can indicate their capability to hold cells suspensions, and their possible support in cell growth and proliferation. Hence, water uptake of the various gels was evaluated by swell test in PBS.

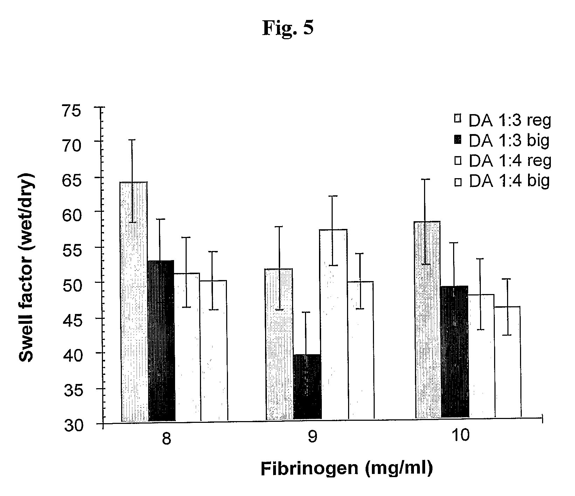

As is evident from Fig. 5, smaller pores increase liquid absorbance. Smaller particles pack less effectively; therefore, the space emptied as a result of the particles removal is bigger. In addition, water is held stronger in smaller pores due to surface and possible capillary forces. Another trend that seemingly can be pointed out, despite the apparent scatter of the measured values, is the increase of swelling with increasing PEG-DA concentration; assumingly due to the tighter hydrogel mesh resulting from higher crosslinker concentration.

### Example 5. Mechanical properties of hydrogel sponges -Tensile properties

Tensile properties of the porous hydrogels were evaluated for wet scaffolds (~7 x 15 x mm³) under constant strain rate of 0.1 mm/s until failure was reached.

A general behavior is common to all samples; the increase in tensile stress up to failure is interrupted by abrupt decreases due to local and limited damage until total failure occurs. This mechanical behavior is typical to composite/nonhomogenous materials and especially to sponge structures where the pores serve both as "hot spots" for damage localization but also as crack arrest elements. As a matter of fact, any apparent stress decrease is not a real one, since after any partial failure the force is applied to a smaller cross section area. Hence, a measurement with compensation to the cross section reduction would have shown a monotonic increase in the tensile stress up to total failure. It should be noted that the data is significantly dispersed, as indicated in differences of roughly tens of percents for samples of similar composition, which introduces a difficulty in analyzing the data. However, such dispersion is a common feature of composite materials and sponges that can be regarded as a composite of polymer and air. Moreover, in addition to the inherent inhomogeniety and inconsistent structure of the hydrogel sponges, there are fluctuations in the samples thickness that cannot be measured properly by mechanical means. Also, it is impossible to grip the samples exactly parallel to the machine axis for allowing a pure unidirectional tension.

Yet, tensile tests under constant strain rate were found to be more adequate to characterize the mechanical properties of the hydrogel sponges despite the data spread. Therefore, such tests were conducted for all of the salt leached hydrogel compositions and structures, aiming to correlate some cell culture features with the scaffolds mechanical properties.

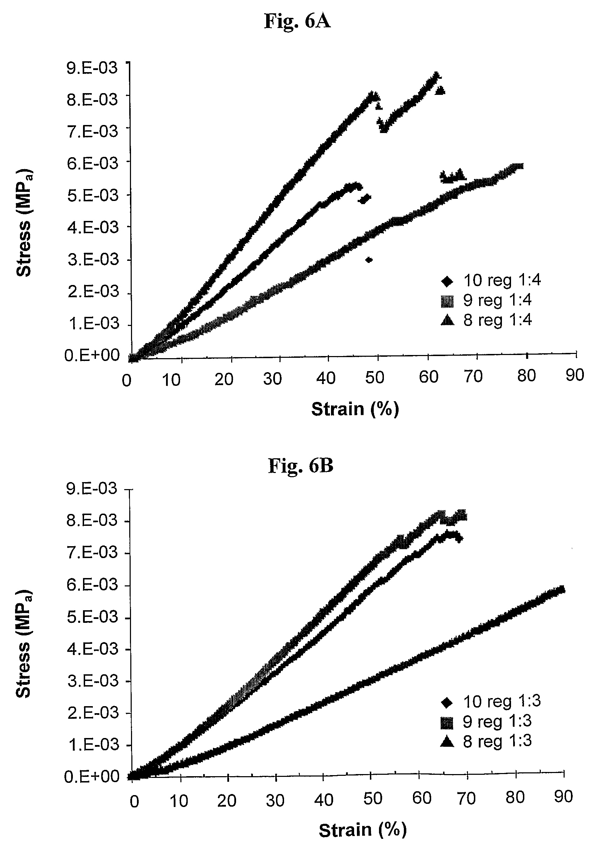

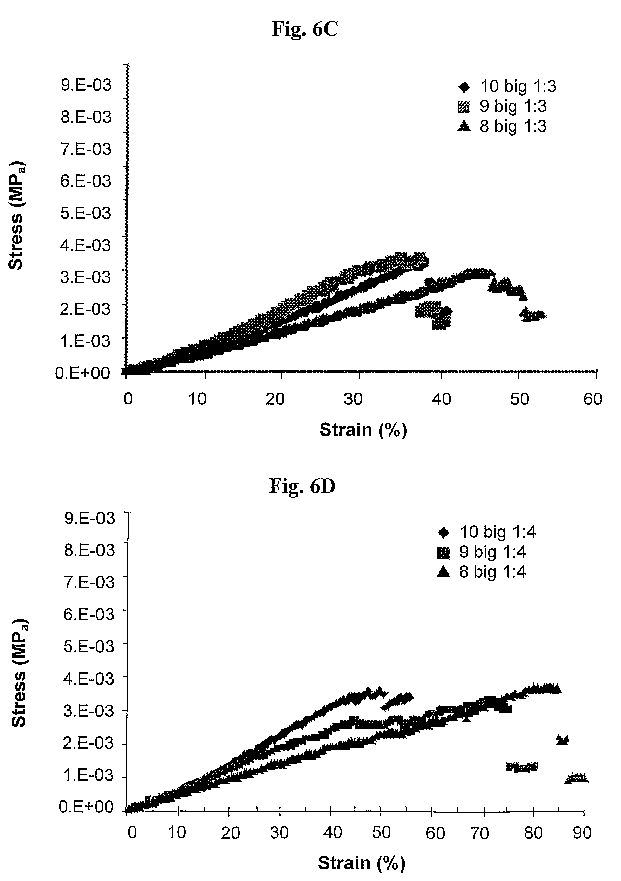

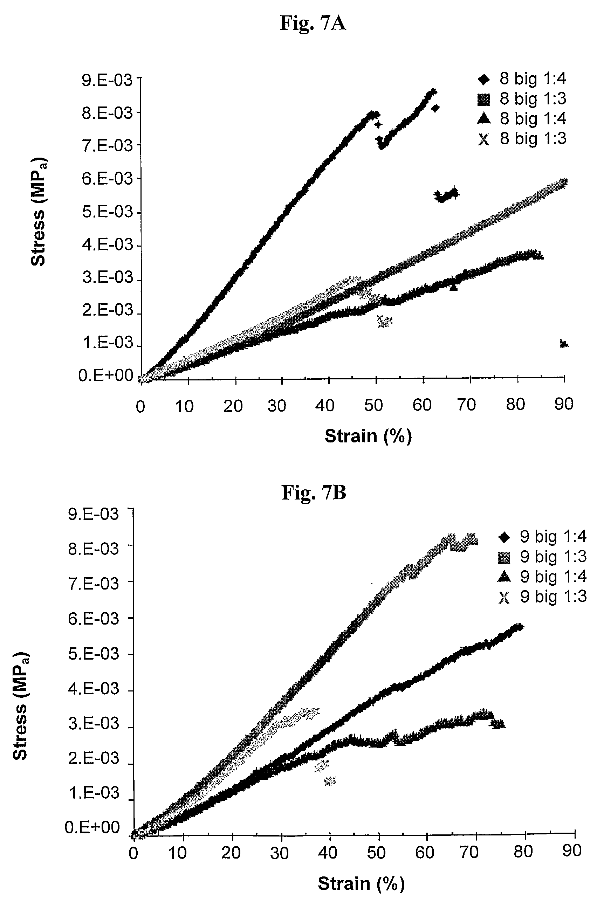

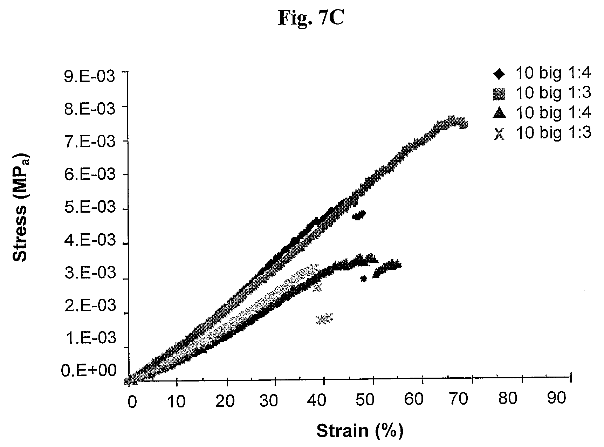

Three parameters were analyzed for their effect on the mechanical properties of the sponge hydrogels; pore size, crosslinker and protein concentration. Inspecting the influence of one parameter at a time, enabled better understanding the effect of each parameter. The sponge hydrogels tensile strength is in the order of few kPa, and the strain to failure is in the order of several tens of percents. The protein concentration seems to have no influence on the tensile properties (Figs. 6A-D and 7A-C). This is reasonable concerning the extremely high concentration of PEG-DA in the hydrogels, and the very low actual protein concentration.

Different from that, a consistent effect of pore size and PEG-DA concentration can be easily noticed; fine pores and higher PEG-DA concentration increase tensile strength.

### Example 6. Proteolytic digestion of hydrogel sponges

Aiming to study the parameters affecting the co-cultured cells proliferation and organization, proteolytic degradation was characterized with regard to protein (fibrinogen) and PEG-DA concentration as well as pore size. As is evident from Fig. 8, the higher the protein concentration the faster the proteolytic degradation. However, PEG-DA concentration and pore size do not affect the degradation rate significantly.

A more thorough insight revealed some influence of PEG-DA concentration on degradation rate, as the lower PEG-DA concentration samples degraded somewhat faster. In the 10 mg/ml PEG-Fib samples, regardless of the pore size, the 1:4 PEG-DA compositions degraded faster than the 1:3 ones. Such trend cannot be found in the 8 mg/ml PEG-Fib samples. No consistency was found for pore size influence; although one can assume that the porous structure enables biodegradability of the said compositions in the first place.

The results described above imply that the very high PEG-DA concentration governs the biodegradability of the sponge hydrogels.

### Example 7. Adhesion properties of hydrogel sponges

Different parameters of the sponge hydrogels affect cell culture behavior, and some can impose even opposing effects. Therefore isolating the physical effect of the porous structure from the chemical parameters was attempted. The chemical properties of the studied compositions were altered by two parameters, fibrinogen (in the PEG-fibrinogen) and PEG-DA concentration. As can be seen from Figs. 9A-C, cells do not adhere well to the gel surface of the compositions in interest. Nevertheless, the PEG-DA concentration makes a major difference as no vital cells were found on the composition of 350:1 molar ratio of PEG to fibrinogen in PEG-Fib/ PEG-DA while some colonies and spreading were found on the hydrogel sponges consisting of 450:1 molar ratio of PEG to fibrinogen in PEG-Fib/ PEG-DA. The poor viability, spreading and proliferation on any of the gels surface, emphasizes the important role of the porous structure in the hydrogel sponges.

### Example 8. Coculture organization on 3D porous hydrogel sponges

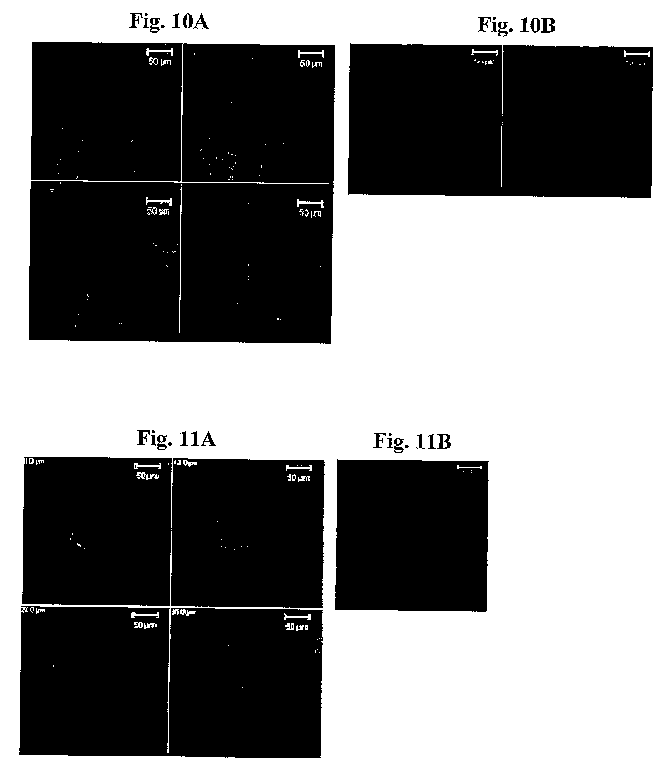

To study the effect of scaffold chemical, physical and mechanical properties as well as its microstructure on tubular- or vessel-like network structure formation in 3D, we first used a co-culture of endothelial cells and fibroblasts as a model. Hence, the effects of several compositions and geometry parameters of sponge hydrogels were studied with regard to organization and differentiation of HUVEC co-cultured with human HFF.
***a***. ***Lyophilized hydrogels***. PEG-Fib gel has been already proven to be adequate for cell culture (Dikovsky et al., 2006; Zoldan et al., 2006), thus it served in the present study as a reference composition in its lyophilized sponge state. This sponge was seeded with HUVEC and HFF cells in ratio 6:1, respectively. Some organization of HUVEC was seen already after 2 days in culture (Fig. 10A), but following 9 days, the organization vanished, and no signaling of HUVEC could be noticed (Fig. 10B). Macroscopically, the scaffold has shrunk significantly and turned from roughly 10 mm² into a small sphere of ~2 mm in diameter. Conversely, lyophilized PEG-Fib/PEG-DA (450:1 molar ratio of PEG to fibrinogen in PEG-Fib/PEG-DA) was tested for biofunctionality. Since this scaffold material is highly non-uniform, cells were absent in large area of the scaffold. The cell populated areas were very small, and typically located in the edges of the scaffold, possibly due to higher porosity and/or lower thickness of these parts, allowing better access to oxygen and nutrients. As shown in Fig. 11A, following 9 days in culture there was some sparse organization of cells to some lumen structures. Even following 14 days few colonies were observed in the populated areas but no ordered organization was detected (Fig. 11B).
***b. Salt leached sponge hydrogels -*** Since the lyophilized compositions were unsatisfactory, the salt leached PEG-Fib/PEG-DA (350:1 molar ratio of PEG to fibrinogen in PEG-Fib/PEG-DA) were evaluated for cell differentiation and organization, employing confocal microscopy as well. The sterilization, seeding and culturing procedure was similar to that described above. Samples were fixed following 1, 7 and 14 days in culture. An experimental matrix of three parameters was set, intended to asses the contribution of each parameter to the organization of the co-cultured cells into lumens and capillaries network (Table 1 above). For simplicity, the term "coarse" designates leaching in the presence of 600-850 µm salt particles, and "fine" designates leaching in the presence of to 212-600 µm salt particles.

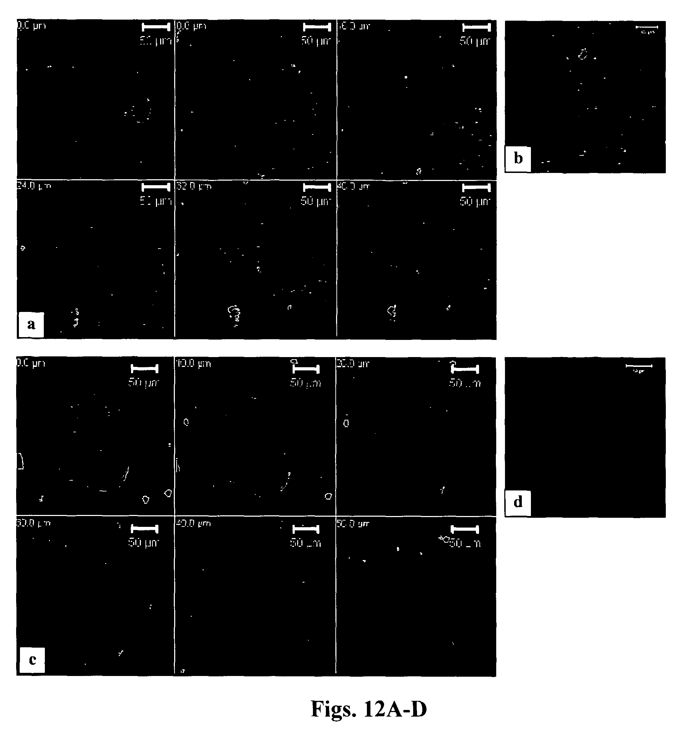

As can be seen from Figs. 12A-D, HUVEC-HFF co-cultures in the sponge hydrogels formed spheroid colonies. Such a form probably stems from the spheroid shape pores of the sponge hydrogel. In sample D (see Table 1) taken after 1 day in culture, the spheroid colonies were separated from each other, exhibiting well-defined borders (Fig. 12A). Of note is the green color at the edges, marking small quantity of HUVEC.

Following 14 days of the hydrogel constructs in culture, most of the colonies remained spheroids (Fig. 12B), but there was a clear organization to full circles of the HUVEC at the edges and the fibroblasts in inner layers (Fig. 12C). Also, there was evidence to differentiation of some of the fibroblasts into smooth muscle cells (SMC), marked in red, lining outside the HUVEC. Moreover, images of these colonies over tens of microns depth revealed a clear tubular structure, i.e., organization into lumens. The image in Fig. 12C looks like a slice from a spherical colony of cells where the fibroblasts form the inner mass and the HUVEC arrange at the outer circle. Moving upward the confocal slices, the inner cell mass fades gradually implying that an elongated tube evolved from the initial cells spheroids (Fig. 12C). Although type D samples consisted mostly of such structures separated from each other, some scarce evidence for spreading of cells was found after 14 days in culture (Fig. 12D).

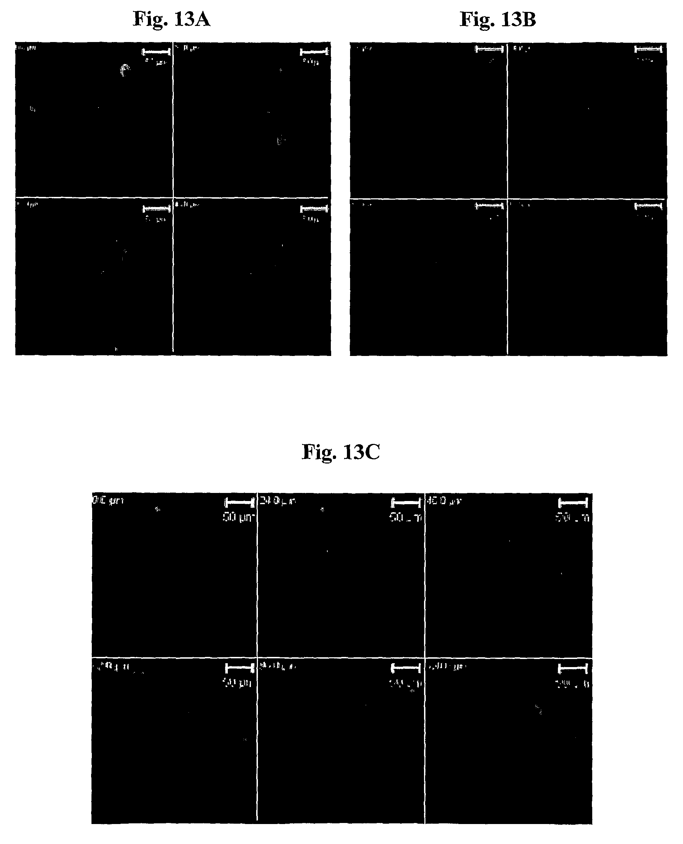

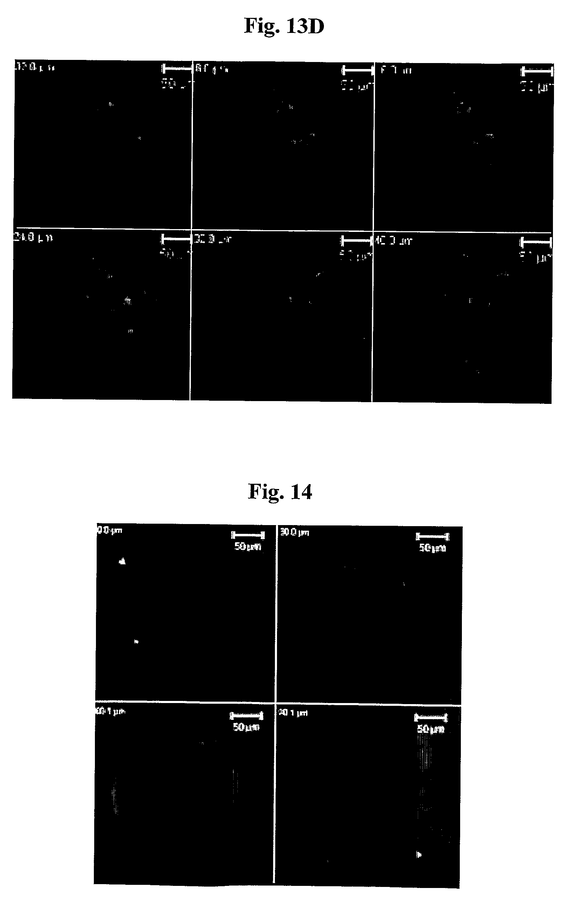

Different from type D samples, B samples consisting of similar composition but bigger pores exhibited organization into core-shell structure and some spreading of the cell colonies already after 1 day in culture (Figs. 13A-B). Following 7 days in culture lumen and elongated spread structures developed as seen in Fig. 13C. The complex structure was spread over 100 µm deep and 200 µm in width and length. These findings suggest that the pore size has a significant effect on HFF-HUVEC organization. Bigger pores are expected to decrease the scaffold modulus, and by that to facilitate cells spreading.

Increasing the fibrinogen concentration to 9 mg/ml in a scaffold of similar PEG-DA concentration and small pores enabled organization of co-cultured cells into tubular structure, and proliferation of cells beyond the lumen defined walls already after 1 day in culture (Fig. 14). This infers the significant effect of protein concentration on cell growth.

The concentration of the crosslinker, PEG-DA, in PBS was shown above to affect the mechanical properties and the water uptake of the scaffolds. Therefore its influence on cell culture was evaluated as well. Based on the results described above, it was hypothesized that the higher PEG-DA concentration (350:1 molar ratio of PEG to fibrinogen in PEG-Fib/PEG-DA) will hinder cell spreading and organization due to the increased stiffness of the scaffold. The expectation was confirmed as depicted in the confocal photographs: after 1 day in culture of C type (8 mg/ml, 350:1 molar ratio of PEG to fibrinogen in PEG-Fib/ PEG-DA, small pores) constructs, only well defined rounded cell colonies were observed (Fig. 15A); after 7 days in culture organization into tubular structures occurred, but the tubular structure was still very well defined, and no spreading of cells or coalescence of colonies was found (Figs. 15B-C). The results described above lead to the conclusion that the least favoring cell spreading set of parameters is lowest protein concentration, highest PEG-DA concentration and small pores. Therefore, it was interesting to study the effect of pore size on a scaffold consisting of highest PEG-DA and lowest fibrinogen concentration. Hence, we investigated constructs of 8 mg/ml, 350:1 molar ratio of PEG to fibrinogen in PEG-Fib/PEG-DA, the same composition, which consisted of big pores architecture (type A).

The type A constructs reflect the pronounced effect of pore size; despite the low protein concentration and high PEG-DA concentration, the co-cultured cells organize into layered lumen like structure.

In summary, salt leached sponge PEG-fibrinogen hydrogels are interesting scaffold materials that support HFF-HUVEC organization into lumen structures. In addition, evolved structures of cells are easily characterized using these scaffolds owing to their full transparency. The mechanical properties and protein concentration seem to have opposing effects on HFF-HUVEC co-cultures; increasing the first hinders proliferation and organization of the cells, while increasing the latter supports them. The effect of protein concentration is assumed to be correlated with proteolytic degradability. Pore size was shown to be a dominant parameter affecting cell culture mainly when the composition parameters are not favored.

### Example 9. Hydrogel sponge comprising 100% crosslinked PEG-DA

120 µl of 20 or 25% PEG-DA aqueous solution was added to 0.4 g of sodium chloride. The mixture was crosslinked by UV irradiation (λ=365 nm) for 5 min. Then the salt was leached in water from the hydrogel, resulting in wet porous/sponge hydrogel. The sponge hydrogel was freezed in (-80)°C for 4 hours and lyophilized overnight resulting in dry sponge. The sponge can be sterilized using UV radiation (λ=365nm) for 20 min prior to application as an implant or as a scaffold for tissue culture.

### REFERENCES

Dikovsky, D., H. Bianco-Peled, and D. Seliktar, The effect of structural alterations of PEG-fibrinogen hydrogel scaffolds on 3-D cellular morphology and cellular migration. Biomaterials, 27(8): p. 1496-506, 2006.
Dikovsky, D., H. Bianco-Peled, and D. Seliktar, Defining the role of matrix compliance and proteolysis in three-dimensional cell spreading and remodeling. Biophys J, 94(7): p. 2914-25, 2008.
Ford, M.C., et al., A macroporous hydrogel for the coculture of neural progenitor and endothelial cells to form functional vascular networks in vivo. Proc Natl Acad Sci U S A, 103(8): p. 2512-7, 2006.
Huang, X., et al., Porous thermoresponsive-co-biodegradable hydrogels as tissue-engineering scaffolds for 3-dimensional in vitro culture of chondrocytes. Tissue Eng, 13(11): p. 2645-52, 2007.
Lee, W.K., et al., Novel poly(ethylene glycol) scaffolds crosslinked by hydrolyzable polyrotaxane for cartilage tissue engineering. J Biomed Mater Res A, 67(4): p. 1087-92, 2003.
Levenberg, S., et al., Engineering vascularized skeletal muscle tissue. Nat Biotechnol, 23(7): p. 879-84, 2005.
Park, J.S., et al., In vitro and in vivo test of PEG/PCL-based hydrogel scaffold for cell delivery application. J Control Release, 124(1-2): p. 51-9, 2007.
Seliktar D. et al, MMP-2 sensitive, VEGF-bearing bioactive hydrogels for promotion of vascular healing. J Biomed Mater Res 68A(40: p. 704-16, 2004.
Zoldan, J. and S. Levenberg, Engineering three-dimensional tissue structures using stem cells. Methods Enzymol, 420: p. 381-91, 2006.
Sannino, A et al., Synthesis and characterization of macroporous poly(ethylene glycol)-based hydrogels for tissue engineering application. J Biomed. Mater Res A 79(2): p. 229-36, 2006.

## Claims

1. A macroporous hydrogel sponge selected from: (i) a synthetic polymer hydrogel sponge, and (ii) a synthetic polymer-polypeptide conjugate hydrogel sponge, said macroporous hydrogel sponge being at least 20% porous and having a pore diameter of 50-1000 µm, wherein said synthetic polymer is crosslinked to an extent determined by effecting the crosslinking of the synthetic polymer or synthetic polymer-polypeptide conjugate in the presence of at least about 30 % by weight crosslinking agent.

2. The macroporous hydrogel sponge of claim 1, wherein the synthetic polymer is a functionalized derivative of a polyalkylene glycol, hydroxyapatite/polycaprolactone (HA/PLC), polyglycolic acid (PGA), poly-L-lactic acid (PLLA), polymethyl methacrylate (PMMA), polyhydroxyalkanoate (PHA), poly-4-hydroxybutyrate (P4HB), polypropylene fumarate (PPF), or polytetrafluoroethylene (PTFE).

3. The macroporous hydrogel sponge of claim 2, wherein the functionalized derivative of a polyalkylene glycol is a linear or branched polyethylene glycol (PEG) functionalized at the terminal ends such as PEG-diacrylate (PEG-DA) or PEG-multiacrylate.

4. The macroporous synthetic polymer-polypeptide conjugate hydrogel sponge of claim 1, wherein the polypeptide is an extracellular matrix protein selected from fibrinogen, fibronectin, vimentin, microtubule-associated protein 1b, neurite outgrowth factor, bacterial cellulose, laminin or gelatin, preferably fibrinogen.

5. The macroporous hydrogel sponge of claim 1, being at least 50 % porous, wherein at least 20 % of said pores have a pore diameter of 50-1000, preferably, 100-600 µm.

6. The macroporous hydrogel sponge of claim 1, wherein crosslinking is effected in the presence of at least about 40 %, 50%, 60%, 70%, 80%, 90%, or 95% by weight crosslinking agent.

7. The macroporous hydrogel sponge of claim 1, wherein the crosslinking agent is a synthetic polymer selected from PEG-DA, PEG-multiacrylate or PEG-vinylsulfone (VS).

8. The macroporous hydrogel sponge of claim 1, wherein the synthetic polymer and the crosslinking agent are identical, preferably both are PEG-DA, and in the synthetic polymer-polypeptide conjugate the polypeptide is fibrinogen.

9. The macroporous hydrogel sponge of any one of claim 1 to 8, prepared by the salt leaching technique, preferably in dry form.

10. The macroporous hydrogel sponge of claim 9, further comprising cells or one or more nonreactive agents attached to, coated on, embedded or impregnated in the sponge, wherein said or one or more nonreactive agents are pharmaceutical or non-pharmaceutical agents selected from disinfectants, chemotherapeutics, antimicrobial agents, antiviral agents, hemostatics, antiphlogistics, anesthetics, analgesics, nutritional supplements, plasma derivatives, enzymes, and proteins such as extracellular matrix proteins, cell adhesion proteins, growth factors, cytokines, hormones, proteases and protease substrates.

11. A cosmetic or pharmaceutical composition comprising a macroporous hydrogel sponge according to any one of claims 1 to 10, preferably a PEG-DA hydrogel sponge or a PEG-DA-fibrinogen conjugate hydrogel sponge.

12. The cosmetic or pharmaceutical composition of claim 11, comprising the hydrogel sponge in dry form suitable for implantation or for injection after wetting.

13. The macroporous hydrogel sponge of claim 1 or a cosmetic or pharmaceutical composition of claim 11 or 12, for use as scaffolds in tissue engineering for tissue repair when said sponge is a synthetic polymer-polypeptide conjugate hydrogel sponge, or for blocking blood vessels when said sponge is a synthetic polymer hydrogel sponge.

14. An aqueous hydrogel-forming composition for producing a macroporous hydrogel sponge of any one of claims 1 to 10, comprising a mixture of:
(i) a synthetic polymer or a synthetic polymer- polypeptide conjugate;
(ii) a cross-linking agent; and
(iii) a salt porogen;
wherein the hydrogel forming-composition comprises at least 30 % by weight of said cross-linking agent.

15. A method of producing a synthetic polymer or a synthetic polymer-polypeptide conjugate macroporous hydrogel sponge of claim 1, said method comprising the steps:
(a) reacting an aqueous solution comprising a synthetic polymer or a synthetic polymer-polypeptide conjugate and a crosslinking agent in the presence of a salt porogen, and subjecting the mixture to crosslinking to thereby produce a hydrogel/porogen composite; and
(b) extracting said porogen from said composite by leaching in water to thereby produce the synthetic polymer hydrogel sponge or the synthetic polymer-polypeptide conjugate hydrogel sponge that, if desired, is further converted to the dry form.

## Patentansprüche

1. Makroporöser Hydrogelschwamm, ausgewählt aus: (i) einem synthetischen Polymerhydrogelschwamm und (ii) einem synthetischen Polymer-Polypeptid-Konjugat-Hydrogelschwamm, wobei der makroporöse Hydrogelschwamm zu zumindest 20 % porös ist und einen Porendurchmesser von 50 bis 1000 µm aufweist, wobei das synthetische Polymer in einem Ausmaß vernetzt ist, das durch Bewirken der Vernetzung des synthetischen Polymers oder synthetischen Polymer-Polypeptid-Konjugats in Gegenwart von zumindest ungefähr 30 Gew.-% Vernetzungsmittel ermittelt wird.

2. Makroporöser Hydrogelschwamm nach Anspruch 1, wobei das synthetische Polymer ein funktionalisiertes Derivat von Polyalkylenglykol, Hydroxyapatit/Polycaprolacton (HA/PLC), Polyglykolsäure (PGA), Poly-L-milchsäure (PLLA), Polymethylmethacrylat (PMMA), Polyhydroxylalkanoat (PHA), Poly-4-hydroxybutyrat (P4HB), Polypropylenfumarat (PPF) oder Polytetrafluorethylen (PTFE) ist.

3. Makroporöser Hydrogelschwamm nach Anspruch 2, wobei das funktionalisierte Derivat eines Polyalkylenglykols ein lineares oder verzweigtes Polyethylenglykol (PEG) ist, das an den terminalen Enden funktionalisiert ist, wie z. B. PEG-Diacrylat (PEG-DA) oder PEG-Multiacrylat.

4. Makroporöser synthetischer Polymer-Polypeptid-Konjugat-Hydrogelschwamm nach Anspruch 1, wobei das Polypeptid ein Protein extrazellulärer Matrix ist, ausgewählt aus Fibrinogen, Fibronektin, Vimentin, mikrotubuliassoziiertem Protein Ib, Neuriten-Auswuchsfaktor, bakterieller Zellulose, Laminin oder Gelatine, vorzugsweise Fibrinogen.

5. Makroporöser Hydrogelschwamm nach Anspruch 1, der zumindest zu 50 % porös ist, wobei zumindest 20 % der Poren einen Porendurchmesser von 50 bis 1000 µm, vorzugsweise 100 bis 600 µm, aufweisen.

6. Makroporöser Hydrogelschwamm nach Anspruch 1, wobei die Vernetzung in Gegenwart von zumindest ungefähr 40 Gew.-%, 50 Gew.-%, 60 Gew.-%, 70 Gew.-%, 80 Gew.-%, 90 Gew.-% oder 95 Gew.-% Vernetzungsmittel bewirkt wird.

7. Makroporöser Hydrogelschwamm nach Anspruch 1, wobei das Vernetzungsmittel ein synthetisches Polymer ist, ausgewählt aus PEG-DA, PEG-Multiacrylat oder PEG-Vinylsulfon (VS).

8. Makroporöser Hydrogelschwamm nach Anspruch 1, wobei das synthetische Polymer und das Vernetzungsmittel identisch sind, wobei vorzugsweise beide PEG-DA sind, und wobei das Polypeptid im synthetischen Polymer-Polypeptid-Konjugat Fibrinogen ist.

9. Makroporöser Hydrogelschwamm nach einem der Ansprüche 1 bis 8, der gemäß der Salzauslaugetechnik hergestellt wurde, vorzugsweise in trockener Form.

10. Makroporöser Hydrogelschwamm nach Anspruch 9, der des Weiteren Zellen oder ein oder mehrere nicht-reaktive Mittel umfasst, die am Schwamm angebunden, auf diesen beschichtet, in diesen eingebettet oder in diesen imprägniert sind, wobei das eine oder die mehreren nicht-reaktiven Mittel pharmazeutische oder nicht-pharmazeutische Mittel sind, ausgewählt aus Desinfektionsmitteln, chemotherapeutischen Mitteln, antimikrobiellen Mitteln, antiviralen Mitteln, Hämostatika, Antiphlogistika, Anästhetika, Analgetika, Nahrungsergänzungsmitteln, Plasmaderivaten, Enzymen und Proteinen wie z. B. Proteinen extrazellulärer Matrix, Zelladhäsionsproteinen, Wachstumsfaktoren, Zytokinen, Hormonen, Proteasen und Proteasesubstraten.

11. Kosmetische oder pharmazeutische Zusammensetzung, die einen makroporösen Hydrogelschwamm nach einem der Ansprüche 1 bis 10 umfasst, vorzugsweise einen PEG-DA-Hydrogelschwamm oder einen PEG-DA-Fibrinogen-Konjugat-Hydrogelschwamm.

12. Kosmetische oder pharmazeutische Zusammensetzung nach Anspruch 11, die den Hydrogelschwamm in trockener Form umfasst, die sich für eine Implantation oder für eine Injektion nach Benetzung eignet.

13. Makroporöser Hydrogelschwamm nach Anspruch 1 oder eine kosmetische oder pharmazeutische Zusammensetzung nach Anspruch 11 oder 12 zur Verwendung als Gerüste beim Gewebe-Engineering zur Gewebereparatur, wenn der Schwamm ein synthetischer Polymer-Polypeptid-Konjugat-Hydrogelschwamm ist, oder zur Blockade von Blutgefäßen, wenn der Schwamm ein synthetischer Polymerhydrogelschwamm ist.

14. Wässrige hydrogelbildende Zusammensetzung zur Herstellung eines makroporösen Hydrogelschwamms nach einem der Ansprüche 1 bis 10, umfassend eine Mischung aus:
(i) einem synthetischen Polymer oder einem synthetischen Polymer-Polypeptid-Konjugat;
(ii) einem Vernetzungsmittel; und
(iii) einem Salzporogen;
wobei die hydrogelbildende Zusammensetzung zumindest 30 Gew.-% des Vernetzungsmittels umfasst.

15. Verfahren zum Herstellen eines makroporösen synthetischen Polymer- oder eines makroporösen synthetischen Polymer-Polypeptid-Konjugat-Hydrogelschwamms nach Anspruch 1, wobei das Verfahren die Schritte umfasst:
(a) Reagieren einer wässrigen Lösung, die ein synthetisches Polymer oder ein synthetisches Polymer-Polypeptid-Konjugat und ein Vernetzungsmittel umfasst, in Gegenwart eines Salzporogens und Aussetzen der Mischung gegenüber einer Vernetzung, um einen Hydrogel-Porogen-Verbundstoff herzustellen; und
(b) Extrahieren des Porogens aus dem Verbundstoff durch Auslaugen in Wasser, um den synthetischen Polymerhydrogelschwamm oder den synthetischen Polymer-Polypeptid-Konjugat-Hydrogelschwamm herzustellen, der nach Wunsch weiter zur trockenen Form umgewandelt wird.

## Revendications

1. Eponge d'hydrogel macroporeuse choisie parmi : (i) une éponge d'hydrogel de polymère de synthèse, et (ii) une éponge d'hydrogel de conjugué polymère-polypeptide de synthèse, ladite éponge d'hydrogel macroporeuse étant poreuse à au moins 20 % et ayant un diamètre de pore de 50-1000 µm, ledit polymère de synthèse étant réticulé dans une mesure déterminée en effectuant la réticulation du polymère de synthèse ou du conjugué polymère-polypeptide de synthèse en présence d'au moins environ 30 % en poids d'agent réticulant.

2. Eponge d'hydrogel macroporeuse selon la revendication 1, dans laquelle le polymère de synthèse est un dérivé fonctionnalisé d'un polyalkylène glycol, d'hydroxyapatite/polycaprolactone (HA/PLC), d'acide polyglycolique (PGA), d'acide poly-L-lactique (PLLA), de poly(méthacrylate de méthyle) (PMMA), de polyhydroxyalcanoate (PHA), de poly-4-hydroxybutyrate (P4HB), de poly(fumarate de propylène) (PPF) ou de polytétrafluoroéthylène (PTFE).

3. Eponge d'hydrogel macroporeuse selon la revendication 2, dans laquelle le dérivé fonctionnalisé d'un polyalkylène glycol est un polyéthylène glycol (PEG) linéaire ou ramifié, fonctionnalisé aux extrémités terminales, tel que le PEG-diacrylate (PEG-DA) ou le PEG-multiacrylate.

4. Eponge d'hydrogel de conjugué polymère-polypeptide de synthèse, macroporeuse, selon la revendication 1, dans laquelle le polypeptide est une protéine de matrice extracellulaire choisie parmi le fibrinogène, la fibronectine, la vimentine, la protéine 1b associée aux microtubules, le facteur d'excroissance des neurites, la cellulose bactérienne, la laminine ou la gélatine, de préférence le fibrinogène.

5. Eponge d'hydrogel macroporeuse selon la revendication 1, étant poreuse à au moins 50 %, au moins 20 % desdits pores ayant un diamètre de pore de 50-1000, de préférence, 100-600 µm.

6. Eponge d'hydrogel macroporeuse selon la revendication 1, dans laquelle la réticulation est effectuée en présence d'au moins environ 40 %, 50 %, 60 %, 70 %, 80 %, 90 % ou 95 % en poids d'agent réticulant.

7. Eponge d'hydrogel macroporeuse selon la revendication 1, dans laquelle l'agent réticulant est un polymère de synthèse choisi parmi le PEG-DA, le PEG-multiacrylate ou la PEG-vinylsulfone (VS).

8. Eponge d'hydrogel macroporeuse selon la revendication 1, dans laquelle le polymère de synthèse et l'agent réticulant sont identiques, de préférence les deux sont le PEG-DA, et, dans le conjugué polymère-polypeptide de synthèse, le polypeptide est le fibrinogène.

9. Eponge d'hydrogel macroporeuse selon l'une quelconque des revendications 1 à 8, préparée par la technique de lessivage au sel, de préférence sous forme sèche.

10. Eponge d'hydrogel macroporeuse selon la revendication 9, comprenant en outre des cellules ou un ou plusieurs agents non réactifs attachés à, appliqués en revêtement sur, noyés ou imprégnés dans l'éponge, ledit ou lesdits agents non réactifs étant des agents pharmaceutiques ou non pharmaceutiques choisis parmi les désinfectants, les agents chimiothérapeutiques, les agents antimicrobiens, les agents antiviraux, les hémostatiques, les antiphlogistiques, les anesthésiques, les analgésiques, les suppléments nutritionnels, les dérivés plasmatiques, les enzymes et les protéines telles que les protéines de matrice extracellulaire, les protéines d'adhésion cellulaire, les facteurs de croissance, les cytokines, les hormones, les protéases et les substrats de protéases.

11. Composition cosmétique ou pharmaceutique comprenant une éponge d'hydrogel macroporeuse selon l'une quelconque des revendications 1 à 10, de préférence une éponge d'hydrogel de PEG-DA ou une éponge d'hydrogel de conjugué PEG-DA-fibrinogène.

12. Composition cosmétique ou pharmaceutique selon la revendication 11, comprenant l'éponge d'hydrogel sous forme sèche appropriée pour une implantation ou pour une injection après mouillage.

13. Eponge d'hydrogel macroporeuse selon la revendication 1 ou composition cosmétique ou pharmaceutique selon l'une des revendications 11 ou 12, destinée à être utilisée en tant qu'échafaudages en ingénierie tissulaire pour une réparation tissulaire lorsque ladite éponge est une éponge d'hydrogel de conjugué polymère-polypeptide de synthèse, ou pour bloquer des vaisseaux sanguins lorsque ladite éponge est une éponge d'hydrogel de polymère de synthèse.

14. Composition aqueuse de formation d'hydrogel destinée à produire une éponge d'hydrogel macroporeuse selon l'une quelconque des revendications 1 à 10, comprenant un mélange de :
(i) un polymère de synthèse ou un conjugué polymère-polypeptide de synthèse ;
(ii) un agent réticulant ; et
(iii) un porogène sel ;
la composition de formation d'hydrogel comprenant au moins 30 % en poids dudit agent réticulant.

15. Procédé de production d'une éponge d'hydrogel macroporeuse de polymère de synthèse ou de conjugué polymère-polypeptide de synthèse, selon la revendication 1, ledit procédé comprenant les étapes :
(a) faire réagir une solution aqueuse comprenant un polymère de synthèse ou un conjugué polymère-polypeptide de synthèse et un agent réticulant en présence d'un porogène sel, et soumettre le mélange à une réticulation pour produire de cette façon un composite hydrogel/porogène ; et
(b) extraire ledit porogène à partir dudit composite par lessivage dans l'eau pour produire de cette façon l'éponge d'hydrogel de polymère de synthèse ou l'éponge d'hydrogel de conjugué polymère-polypeptide de synthèse qui, si souhaité, est encore convertie en la forme sèche.
